# EUROPEAN PATENT APPLICATION

(11) **EP 4 541 263 A1**
(43) Date of publication of application: **23.04.2025**
(21) Application number: 23824277.0
(22) Date of filing: 16.06.2023
(51) Int. Cl.: A61B 5/00, A61B 5/15, A61M 5/158, A61B 5/145, A61B 5/155

(54) **INSERTION APPARATUS FOR ANALYTE MONITORING APPARATUS**

(30) Priority: 17.06.2022 KR 20220074384; 24.06.2022 KR 20220077473; 24.06.2022 KR 20220077474; 15.07.2022 KR 20220087608; 02.12.2022 KR 20220166655; 02.12.2022 KR 20220166656; 02.12.2022 KR 20220166657; 04.01.2023 KR 20230001205; 14.06.2023 KR 20230076324
(71) Applicant: SD Biosensor, Inc., Gyeonggi-do 16690 (KR)
(72) Inventor: LEE, Hyo Keun, Suwon-si, Gyeonggi-do 16690 (KR); PARK, Hyo-Lim, Suwon-si, Gyeonggi-do 16684 (KR); LEE, Won-Chul, Gimhae-si, Gyeongsangnam-do 50990 (KR); LEE, Jun Hwang, Yongin-si, Gyeonggi-do 16999 (KR); HONG, Soonmin, Hwaseong-si, Gyeonggi-do 18477 (KR)
(74) Representative: J A Kemp LLP
(86) International application number: PCT/KR2023/008346
(87) International publication number: WO 2023/244064

(57) **Abstract**

Provided, according to one embodiment of the present disclosure, is an applicator comprising: a hollow column-shaped housing having an opening formed in one surface thereof; a first button disposed on the upper surface of the housing; a second button disposed on the outer peripheral surface of the housing; a needle carrier configured to be coupled to the second button and undergoing a linear motion in between a distal portion and a proximal portion inside the housing; and a drive part configured to be coupled to the first button and provide power for the linear motion of the needle carrier.

## Description

### Technical Field

The present invention relates to an insertion apparatus for an analyte monitoring apparatus, and more particularly, to a detailed structure of an insertion apparatus for an apparatus for monitoring body analytes.

### Background Art

Contents described in this section merely provide background information about the present disclosure and do not constitute prior art.

As preference for processed foods has recently increased, people are easily exposed to monosaccharides. Due to this environment, diabetes is also increasing. When blood glucose level drops rapidly or rises rapidly, diabetic patients may go into shock, and in rare cases, it can lead to death. Thus, diabetic patients are required to monitor their blood glucose levels continuously.

In a blood glucose measuring device according to the related art, a lancet is used to make an incision at the end of the finger, and the diabetic patient's blood that flows through the incision is inserted into a blood glucose analyzing device, thereby calculating their blood glucose level.

This type of blood glucose measuring device causes pain and fear to a user. In addition, it is realistically difficult for the patient to cut their own finger every hour and make it bleed.

In order to solve this problem, a continuous glucose monitoring (CGM) system has been developed. When a sensor having a fine thickness and an electronic device (hereinafter, a "transmitter") for analyzing an analyte (hereinafter, "blood glucose") measured by the sensor are attached to the diabetic patient's skin, blood glucose levels are continuously measured for one week to ten days.

The sensor of the transmitter is so thin that it is difficult to directly penetrate the skin. Thus, a configuration (hereinafter, an "applicator") in which a needle is inserted into the skin, a sensor is inserted into the gap and the needle is drawn out, is required.

It is significant to provide a soft feel when the applicator is used.

Meanwhile, according to US 2021-0038131 A1 document, a sensor introducer 106 should be inserted into a sensor inserter 500. In this case, the sensor introducer needs to be inserted into the sensor inserter by adjusting its direction of the sensor introducer, and in this procedure, there is a high risk that a user will feel fatigued. Thus, the applicator is desirable to operate with easy operation.

In addition, since the needle is inserted directly into the user's skin, the applicator needs to be used only once for hygiene purposes. Since the high manufacturing cost of disposable products can be a financial burden on the user, it is desirable to reduce the manufacturing cost as much as possible.

In addition, since the needle is inserted directly into the user's skin, it is very significant to prevent reuse. Thus, it is desirable to provide an applicator with a structure capable of preventing reuse structurally.

In addition, when the applicator is used, in order to minimize pain, it is desirable that an operation of inserting and drawing out the needle is performed as quickly as possible.

In addition, since there is a risk that some users do not read a user manual closely, it is desirable that a use method is intuitively recognized.

In addition, when the assembled applicator is disassembled due to external impact, the user will not be able to use the normal transmitter. Thus, even when there is external impact, it is desirable to maintain the applicator solidly.

In addition, it is desirable to provide an applicator that can stably fix and support the transmitter inside the applicator and prevent the transmitter from falling out even when there is external impact.

In addition, it is desirable to provide a configuration for preventing accidental firing regardless of the user's intention.

### Detailed Description of the Invention

### Technical Problem

Thus, the present disclosure provides an applicator with a safety device to prevent firing of the applicator due to external impact.

In addition, the present disclosure also provides an applicator to which a transmitter can be stably fixed even when there is external impact.

The problems to be solved by the present invention are not limited to the problems mentioned above, and other unmentioned problems will be clearly understood by those skilled in the art from the description below.

### Technical Solution

According to an embodiment of the present invention, there is provided an applicator including a hollow column-shaped housing having an opening formed in a bottom thereof, a first button disposed on an upper surface of the housing, a second button disposed on an outer circumferential surface of the housing, a needle carrier configured to be fastened to the second button and making a straight line motion between a distal position and a proximal position inside the housing, and a driving portion configured to be fastened to the first button and to provide power of the straight line motion of the needle carrier.

In addition, preferably, the driving portion may include a wound spring in a compressed state coupled to an inner circumferential surface of the housing, a wheel having one surface facing the spring and constrained to rotation of the spring, an arc-shaped firing preventing portion that protrudes from the other surface of the wheel and is formed along a circumference of the wheel, and a guide protrusion that protrudes from the other surface of the wheel and is disposed radially inward from the firing preventing portion, wherein the needle carrier includes a lateral guide, and a longitudinal guide that is formed from one surface of the lateral guide and extends along a lengthwise direction of the housing.

In addition, preferably, the first button may include an extension portion that protrudes from a lower surface of the first button, and an engagement protrusion that protrudes from one surface of the extension portion toward a radial outside of the first button and is configured to face the firing preventing portion, and the engagement protrusion may be spaced apart from the lower surface of the first button, and when the first button is pressed, the firing preventing portion may be disposed in the spaced space between the engagement protrusion and the lower surface of the first button.

In addition, preferably, the needle carrier may include a hook protrusion that protrudes from a lateral surface of the longitudinal guide, and the second button may include an extension portion that protrudes from a lower surface of the second button and disposed toward a radially inside of the housing, and a hook that protrudes from one surface of the extension portion toward a radial inside of the second button and configured to face the hook protrusion, and the hook may be spaced apart from the lower surface of the second button, and when the second button is pressed, the hook protrusion may be disposed in the spaced space between the hook and the lower surface of the second button.

In addition, preferably, the first button may be configured to limit rotation of the driving portion, the second button may be configured to limit a straight line motion of the needle carrier, and when the first button and the second button are simultaneously or sequentially pressed, the driving portion may be rotated so that the needle carrier moves from the distal position to the proximal position.

In addition, preferably, the applicator may further include a transmitter holder that is pressed by the needle carrier, which moves from the distal position to the proximal position and is configured to hold a transmitter, wherein the transmitter holder includes a transmitter support portion configured to surround at least a part of the transmitter, and a pusher rotatably mounted on an upper surface of the transmitter holder.

In addition, preferably, one end of the pusher may be disposed outside the transmitter holder, the other end of the pusher may be disposed inside the transmitter holder, and one end of the pusher may protrude from a lower surface of the transmitter holder.

In addition, preferably, when the transmitter holder moves from the distal position to the proximal position by the needle carrier, one end of the pusher may be in contact with an inner bottom of the housing, and the other end of the pusher may press an upper surface of the transmitter.

In addition, preferably, the transmitter support portion and the transmitter may be frictionally coupled to each other.

In addition, according to another embodiment of the present disclosure, there is provided a sensor insertion apparatus including a sensor of which at least a part is inserted into a body and which is configured to detect body analytes, a transmitter configured to process information related to the body analytes obtained from the sensor, and an applicator configured to move the sensor and the transmitter from a proximal position to a distal position, wherein the applicator includes a hollow column-shaped housing having an opening formed at the bottom thereof, a first button disposed on an upper surface of the housing, a second button disposed on an outer circumferential surface of the housing, a needle carrier configured to be fastened to the second button and making a straight line motion between a distal position and a proximal position inside the housing, and a driving portion configured to be fastened to the first button and to provide power of the straight line motion of the needle carrier.

### Effects of the Invention

As described above, according to the present embodiment, since an applicator is fired only when both a first button and a second button are pressed, firing due to an accident such as external impact or the like can be prevented.

In addition, the first button and the second button can be pressed regardless of the order, which makes the device user-friendly and easy to use.

### Description of the Drawings

FIGS. 1A and 1B are perspective views of an applicator according to a first embodiment of the present disclosure.
FIG. 2 is an exploded perspective view of the applicator according to the first embodiment of the present disclosure.
FIG. 3A is a cross-sectional view of FIG. 1A taken along a line I-I'.
FIG. 3B is a cross-sectional view of FIG. 1A taken along a line II-II'.
FIG. 4 illustrates an operation of an applicator according to the first embodiment of the present disclosure.
FIGS. 5A and 5B provide perspective views of an applicator according to a second embodiment of the present disclosure.
FIG. 6 is an exploded perspective view of the applicator according to the second embodiment of the present disclosure.
FIG. 7 is a cross-sectional view of FIG. 5B taken along a line I-I'.
FIG. 8 illustrates an operation of the applicator according to the second embodiment of the present disclosure.
FIG. 9A is a cross-sectional view of FIG. 5B taken along a line II-II'.
FIG. 9B is a cross-sectional view of FIG. 5B taken along a line III-III'.
FIG. 10 is an enlarged view of the bottom of the applicator according to the second embodiment of the present disclosure.
FIGS. 11A through 11C are perspective views of an applicator according to a third embodiment of the present disclosure.
FIG. 12 is an exploded perspective view of the applicator according to the third embodiment of the present disclosure.
FIGS. 13A and 13B illustrate the combination between internal components of the applicator according to the third embodiment of the present disclosure.
FIG. 14 illustrates a fastening method of a spring according to the third embodiment of the present disclosure.
FIG. 15 illustrates an operation of the applicator according to the third embodiment of the present disclosure.
FIGS. 16A and 16B are perspective views of an applicator according to a fourth embodiment of the present disclosure.
FIG. 17 is an exploded perspective view of the applicator according to the fourth embodiment of the present disclosure.
FIG. 18 is a cross-sectional view of FIG. 16A taken along a line I-I'.
FIG. 19 illustrates an operation of the applicator according to the fourth embodiment of the present disclosure.
FIG. 20 is a cross-sectional view and a partially-enlarged view of FIG. 16A taken along a line II-II'.
FIG. 21 is a cross-sectional view and a partially-enlarged view of FIG. 16A taken along a line III-III'.
FIG. 22 is a perspective view of an applicator according to a 5^{th}-1 embodiment of the present disclosure.
FIG. 23 is an exploded perspective view of the applicator according to the 5^{th}-1 embodiment of the present disclosure.
FIG. 24 illustrates the inside of the applicator according to the 5^{th}-1 embodiment of the present disclosure.
FIG. 25 illustrates an operating principle of the applicator according to the 5^{th}-1 embodiment of the present disclosure.
FIGS. 26 and 27 illustrate a configuration for preventing firing of the applicator according to the 5^{th}-1 embodiment of the present disclosure.
FIG. 28 is a bottom perspective view of the applicator according to the 5^{th}-1 embodiment of the present disclosure.
FIG. 29 illustrates a transmitter holder and a transmitter of the applicator 5^{th}-1 embodiment of the present disclosure.
FIG. 30 illustrates a state after firing of the applicator according to the 5^{th}-1 embodiment of the present disclosure.
FIG. 31 illustrates an operating principle of an applicator according to a 5^{th}-2 embodiment of the present disclosure.
FIG. 32 is a perspective view of an applicator according to a 6^{th}-1 embodiment of the present disclosure.
FIGS. 33A and 33B illustrate the inside of the applicator according to the 6^{th}-1 embodiment of the present disclosure.
FIG. 34 is a cross-sectional view of FIG. 32 taken along a line I-I' and an enlarged view thereof.
FIG. 35 is a perspective view of an applicator according to a 6^{th}-2 embodiment of the present disclosure.
FIG. 36 is a cross-sectional perspective view of the applicator according to the 6^{th}-2 embodiment of the present disclosure.
FIG. 37 is a perspective view of an applicator according to a seventh embodiment of the present disclosure.
FIG. 38 illustrates the inside of the applicator according to the seventh embodiment of the present disclosure.
FIG. 39 is a front view of a needle carrier according to the seventh embodiment of the present disclosure.
FIG. 40 illustrates a configuration for preventing firing of the applicator according to the seventh embodiment of the present disclosure.

### Mode of the Invention

Hereinafter, some embodiments of the present disclosure will be described in detail with reference to the drawings. When giving reference numerals to components in each drawing, it should be noted that identical components are given the same symbols as much as possible even if they are shown on different drawings. In addition, when describing the present disclosure, if a detailed description of a related known configuration or function is judged to obscure the gist of the present disclosure, the detailed description is omitted.

In describing components of embodiments according to the present disclosure, symbols such as first, second, i), ii), a), b), etc. may be used. These symbols are only for distinguishing the components from other components, and the nature and order or sequence of the components is not limited by the symbols. When a part in the specification is said to "include" or "provide" a component, this does not mean that other components are excluded, but rather that other components can be further included, unless explicitly stated otherwise.

In the present disclosure, the term "distal position" means a position that is relatively farther from the skin than the "proximal position". Meanwhile, distal and proximal are concepts of relative positions and can be understood as one element being located proximal to another element while still being distal to others.

In the present disclosure, the term "initial state" means a state before a user uses an applicator.

In addition, in the present disclosure, the term "fire" means a user's behavior by which an applicator operates to insert a needle into the user's skin.

In addition, in the present disclosure, the term "retreat" means a process where the needle returns to its original position after perforating and being inserted into the user's skin.

In addition, in the present disclosure, the term "upward" means a direction from proximal to distal.

In addition, in the present disclosure, the term "downward" means a direction from distal to proximal.

In addition, in the present disclosure, the term "horizontal" refers to a flat surface that the applicator contacts when the applicator is used, and may mean a skin surface. In the present disclosure, the curve of the skin surface has not been considered, and it will be described on the assumption that the user's skin surface is flat.

### 1. First embodiment

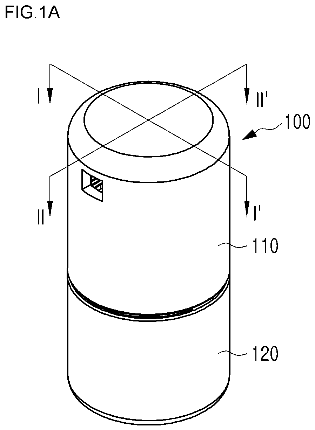

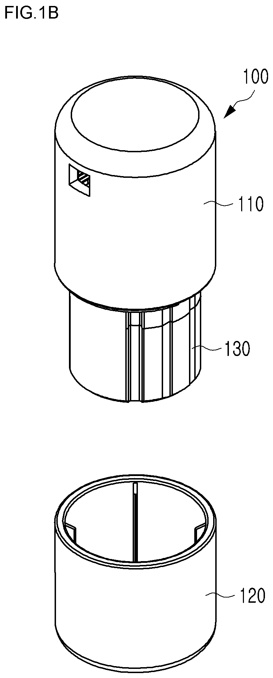

FIGS. 1A and 1B are perspective views of an applicator according to a first embodiment of the present disclosure.

Specifically, FIG. 1A illustrates a state in which a cap 120 is mounted, and FIG. 1B illustrates a state in which the cap 120 is removed.

The applicator 100 according to the first embodiment of the present disclosure includes a handle 110 and a cap 120 configured to be mounted on the handle 110 (see FIG. 1A).

When the cap 120 according to the first embodiment of the present disclosure is removed from the handle 110, a part of a support portion 130 is exposed (see FIG. 1B). The support portion 130 is in direct contact with the user's skin and is formed so that at least a part of the support portion 130 may be inserted into the handle 110.

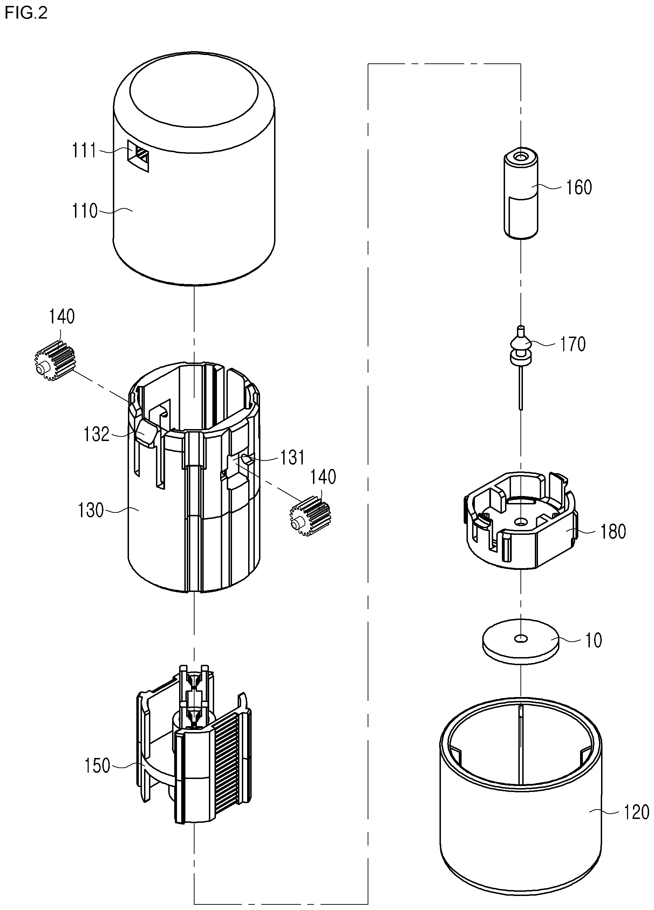

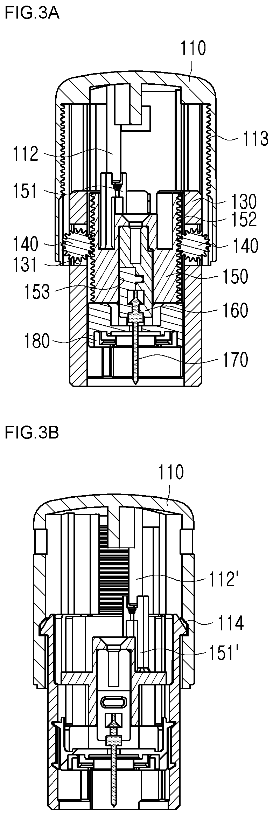

FIG. 2 is an exploded perspective view of an applicator according to the first embodiment of the present disclosure. FIG. 3A is a cross-sectional view of FIG. 1A taken along a line I-I'. FIG. 3B is a cross-sectional view of FIG. 1A taken along a line II-II'.

The combination relationship between components of the applicator 100 according to the first embodiment of the present disclosure will be described with reference to FIGS. 2 to 3B.

The applicator 100 according to the first embodiment includes a handle 110, a cap 120, a support portion 130, one or more gears 140, a needle carrier 150, a needle holder 160, a needle 170, and all or part of a transmitter holder 180.

The handle 110 is configured to be grasped by a user, and preferably, has an approximately cylindrical shape.

The handle 110 includes a hook hole 111, one or more pressing rods 112, a first rack 113, and all or part of a hook mounting groove 114.

The hook hole 111 is formed on an outer circumferential surface of the handle 110 and is configured so that the hook 132 may be hung in the hook hole 111. The hook hole 111 is preferably formed at the top, i.e., above the half of the height of the handle 110.

One or more pressing rods 112 are rods that protrude inwards from an inner ceiling surface of the handle 110 (see FIG. 3A). One or more pressing rods 112 are configured to press the needle carrier 150.

In this case, preferably, one or more pressing rods 112 include a pair of pressing rods 112 and 112' arranged in positions symmetrical to each other with respect to the center of the handle 110. Thus, when the needle carrier 150 is pressed by the handle 110, the needle carrier 150 may be pressed with the same force overall, without being biased to one side. As such, when the needle carrier 150 moves from the distal position to the proximal position or from the proximal position to the distal position again, the needle carrier 150 may move stably, and this may provide a soft feel of use to the user.

The first rack 113 is formed on an inner circumferential surface of the handle 110 along the height direction of the handle 110. The first rack 113 is formed as a pair to face one or more gears 140 (see FIG. 3A).

The hook mounting groove 114 is formed on the inner circumferential surface of the handle 110 and is configured so that the hook 132 may be hung in the hook mounting groove 114. The hook mounting groove 114 is formed at the bottom, i.e., below the half of the height of the handle 110. Thus, in an initial state, the hook 132 may be fixed to the hook mounting groove 114 (see FIG. 3B).

Meanwhile, the hook 132 may be formed to protrude more from the top to the bottom. Also, the hook 132 is configured to have a lower surface parallel to a horizontal plane. The hook mounting groove 114 may be formed to correspond to the shape of the hook 132. Thus, the hook 132 is hung in the hook mounting groove 114 in the initial state so that the needle carrier 150 does not move proximally with respect to the handle 110 anymore.

The cap 120 is configured to cover at least a part of the support portion 130 and may protect the inside of the applicator 100 from external contaminants or impact. The user takes the cap 120 off from the handle 110 so that the user is ready to use the applicator 100.

The support portion 130 is configured so that at least a part of the support portion 130 may be inserted into the handle 100, and as the handle 110 is pressed, the support portion 130 is configured to make a straight line motion inside the handle 110.

The support portion 130 may include a gear hole 131 and a hook 132.

The gear hole 131 is formed so that one or more gears 140 may be mounted, preferably, above the half of the height of the support portion 130, i.e., at the top. One or more gears 140 inside the gear hole 131 may rotate with respect to one axis. The gear hole 131 is formed as a pair, and preferably, a pair of gear holes 131 are arranged to face each other.

The hook 132 protrudes radially outwardly from an outer circumferential surface of the support portion 130. The hook 132 may be hung in the hook mounting groove 114 in an initial state (see FIG. 3B), and the hook 132 may be hung in the hook hole 111 when the handle 110 is pressed all the way.

One or more gears 140 are arranged on the support portion 130 and are configured to rotate along one axis. In this case, one or more gears may be preferably a spur gear, and a pair of gears may be arranged in parallel to each other.

One or more gears 140 may rotate along a part of the handle 110 or along a part of the needle carrier 150. Specifically, one or more gears 140 may be configured to rotate while being engaged with the first rack 113 of the handle 110 or the second rack 152 of the needle carrier 150.

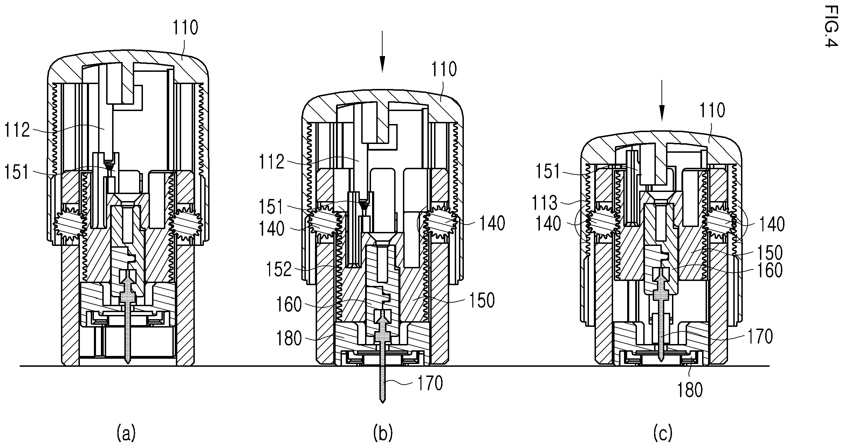

The needle carrier 150 is configured to make a straight line motion inside the support portion 130. The needle carrier 150 may be engaged with one or more gears 140 and may make a straight line motion by rotation of one or more gears 140. With regard to the motion of the needle carrier 150, this will be described in detail with reference to FIG. 4.

The needle carrier 150 includes a rod gripping part 151, a second rack 152, and all or part of a through hole 153.

The rod gripping part 151 is primarily in contact with the pressing rod 112 as the handle 110 is pressed, and is configured to deliver a pressing force of the handle 110 to the needle carrier 150 (see FIG. 3A). In this case, a distance between the handle 110 and the needle carrier 150 may be consistently maintained by the rod gripping part 151. Also, from when the handle 110 is pressed above a certain level, the rod gripping part 151 surrounds at least a part of the outer circumferential surface of the pressing rod 112 and is configured to move along the height direction of the pressing rod 112. Thus, a distance between the handle 110 and the needle carrier 150 may be gradually decreased. With regard to the relationship between the rod gripping part 151 and the pressing rod 112, this will be described in detail with reference to FIG. 4.

It is preferable that the rod gripping part 151 includes a pair of rod gripping parts 151 and 151' arranged in positions symmetrical to each other with respect to the center of the handle 110. In this case, a pair of rod gripping parts 151 and 151' are arranged to face a pair of pressing rods 112 and 112'.

The rod gripping part 151 may have an approximately clamp shaped end so as to wrap the pressing rod 112. However, since it is preferable that, in an initial state, the rod gripping part 151 is pressed by the pressing rod 112 and moves downward, a shortest distance between both ends of a clamp is preferably smaller than a diameter of the pressing rod 112.

The second rack 152 is formed on the outer circumferential surface of the needle carrier 150 along the height direction of the needle carrier 150. The second rack 152 is formed as a pair so as to face one or more gears 140 (see FIG. 3A).

The through hole 153 is formed in the center of the needle carrier 150 and is formed to perforate the needle carrier 150. A needle holder 160 may be pressed in the through hole 153.

The needle holder 160 is constrained to the straight line motion of the needle carrier 160 and is configured to make a straight line motion together. To this end, it is preferable that the needle carrier 160 is frictionally coupled to the through hole 153. However, the present disclosure is not limited thereto, and either adhesive bonding or structural bonding is acceptable.

The needle 170 is coupled to the needle holder 160, is constrained to the straight line motion of the needle holder 160 and is configured to make a straight line motion together. The needle 170 is configured to perforate the user's skin.

The transmitter holder 180 is coupled to the transmitter 10 and is configured to make a straight line motion from the distal position to the proximal position. Meanwhile, the transmitter holder 180 moves only in a direction from the distal position to the proximal position.

FIG. 4 illustrates an operation of an applicator according to a first embodiment of the present disclosure. Meanwhile, in FIG. 4, it will be noted that the transmitter 10 is omitted from the drawing so as to represent the structure of the applicator 100 in more detail.

(a) of FIG. 4 illustrates a state before the handle 110 is pressed, i.e., an initial state. Referring to (a) of FIG. 4, in the initial state, the handle 110, the needle carrier 150, the needle holder 160, the needle 170, and the transmitter holder 180 are arranged in the distal position.

Also, one or more gears 140 are in a state where they face the second rack 152, and do not face the first rack 113.

Also, the rod gripping part 151 is a state in which it is only in contact with the pressing rod 112.

(b) of FIG. 4 illustrates a state in which about the half of the handle 110 is pressed and the needle 170 is inserted into the skin.

Referring to (b) of FIG. 4, when the handle 110 is pressed, the pressing rod 112 presses the rod gripping part 151. In this case, as described above, since a shortest separation distance between both ends of the rod gripping part 151 is smaller than the diameter of the pressing rod 112, a pressing force applied to the handle 110 is delivered to the needle carrier 150, the needle holder 160, and the needle 170 through the rod gripping part 151. In this case, although the rod gripping part 151 is pressed by the pressing rod 112, the pressing rod 112 is not inserted between both ends of the clamp. Thus, in the initial state, a distance between the handle 110 and the needle carrier 150 and a distance between the handle 110 and the needle carrier 150 in a state in which about the half of the handle 110 is pressed, may be the same.

Meanwhile, while the needle carrier 150 moves to the proximal position, one or more gears 140 rotate while being engaged with the second rack 152. In this case, one or more gears 140 rotate in opposite directions, and the gear 140 shown on the left rotates clockwise, and the gear 140 shown on the right rotates counterclockwise.

When the needle 170 is inserted into the user's skin to its maximum depth, it is preferable that one or more gears 140 face the distal end of the second rack 152.

The transmitter holder 180 moves from the distal position to the proximal position together by the movement of the needle carrier 150.

(c) of FIG. 4 illustrates a state in which the handle 110 is pressed to the end.

Referring to (c) of FIG. 4, when the handle is pressed to a certain level or more, the pressing rod 112 may be inserted between both ends of the rod gripping part 151. In this case, the pressing rod 112 may move along the lengthwise direction of the rod gripping part 151, and a distance between the handle 110 and the needle carrier 150 decreases as the handle 110 is pressed.

One or more gears 140 face the first rack 113 and ratate as the first rack 113 moves downward. In this case, one or more gears 140 rotate in an opposite direction to the rotation direction in (b) of FIG. 4. That is, the gear 140 on the left rotates counterclockwise, and the gear 140 on the right rotates clockwise.

While one or more gears 140 rotate, they are engaged with the second rack 152 again. Meanwhile, due to the opposite direction rotation of one or more gears 140, the needle carrier 150 moves from the proximal position to the distal position. Thus, the needle holder 160 and the needle 170 move from the proximal position to the distal position. Thus, the needle 170 may be drawn out from the user's skin.

Meanwhile, the transmitter holder 180 is formed not to be separately fastened to the needle carrier 150. Thus, although the needle carrier 150 moves from the proximal position to the distal position, the transmitter holder 180 may remain in the proximal position.

In the applicator 100 according to the first embodiment of the present disclosure, the needle 170 may be inserted into and drawn out from the skin depending on the combination relationship between gears and racks inside the applicator 100 only with the user's operation of grasping the handle 110 and pressing the handle 110 from the distal position to the proximal position once, so that an operation method is simple, the number of parts is small and manufacturing is easy.

### 2. Second embodiment

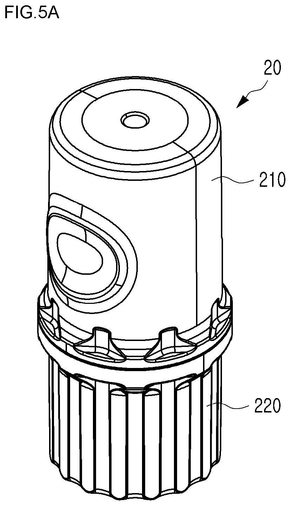

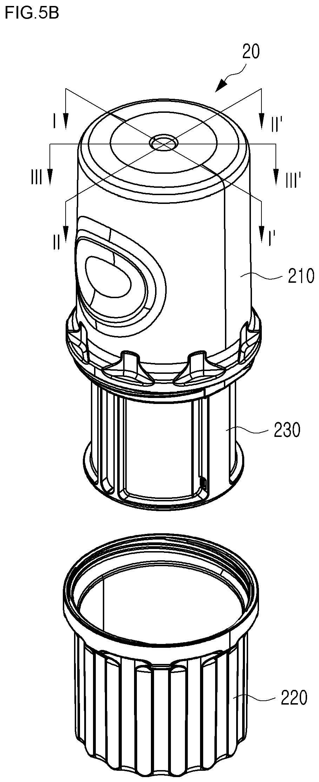

FIGS. 5A and 5B are perspective views of an applicator according to a second embodiment of the present disclosure.

Specifically, FIG. 5A illustrates a state in which a cap 220 is mounted, and FIG. 5B illustrates a state in which the cap 220 is removed.

An applicator 200 according to the second embodiment of the present disclosure includes a handle 210 and the cap 220 configured to be mounted on the handle 210 (see FIG. 5A).

When the cap 220 is removed from the handle 210, all or part of the support portion 230 is exposed (see FIG. 5B). The support portion 230 is in direct contact with the user's skin and is formed so that at least a part of the support portion 230 is inserted into the handle 210.

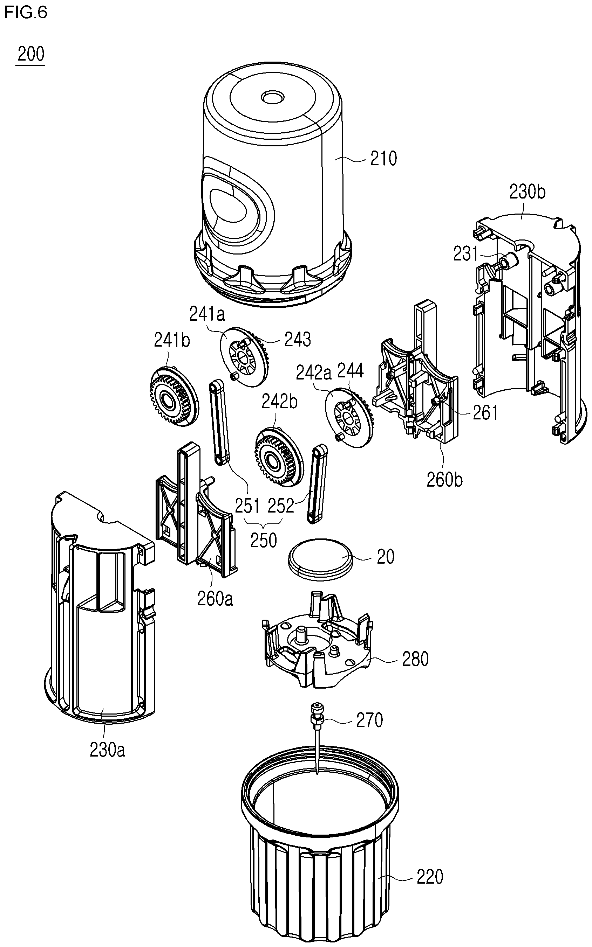

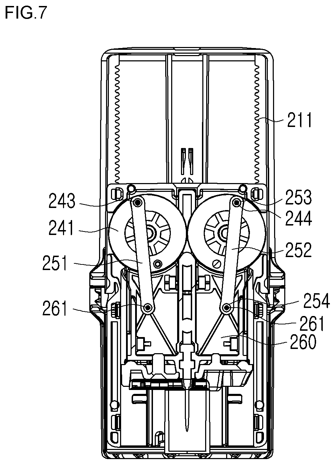

FIG. 6 is an exploded perspective view of an applicator according to the second embodiment of the present disclosure. FIG. 7 is a cross-sectional view of FIG. 5B taken along a line I-I'.

The combination relationship between components of the applicator 200 according to the second embodiment of the present disclosure will be described with reference to FIGS. 6 and 7.

The applicator 200 according to the second embodiment includes a handle 210, a support portion 230, one or more cranks 240, one or more connecting rods 250, a needle carrier 260, a needle 270, a transmitter holder 280, and all or part of a cap 220.

The handle 210 is configured to be grasped by a user, and preferably, has an approximately cylindrical shape.

The support portion 230 is configured so that at least a part of the support portion 230 is inserted into the handle 210, and as the handle 210 is pressed, the support portion 230 is configured to make a straight line motion inside the handle 210.

Preferably, the support portion 230 may be manufactured by injection molding. In this case, a first configuration 230a and a second configuration 230b may be individually manufactured, and the first configuration 230a and the second configuration 230b may be assembled with each other, thereby forming the support portion 230.

The support portion 230 includes a crank shaft 231 that protrudes radially inward from one surface. One or more cranks 240 may be coupled to the crank shaft 231.

One or more cranks 240 are configured to make a rotation motion or a straight line motion inside the handle 210. Meanwhile, in the present disclosure, it has been described on the assumption that there are two cranks 240, however, one crank or three or more cranks may be present, and it will be noted that this may be properly design-changed by a designer.

One or more cranks 240 include a first crank 241 and a second crank 242. The first crank 241 and the second crank 242 may be manufactured by injection molding, preferably. In this case, each crank may be formed by assembling first assemblies 241a and 242a and second assemblies 241b and 242b with each other.

One or more cranks 240 may make a rotation motion along a rack (see 211 of FIG. 7) formed inside the handle 210. The rack 211 may be formed on an inner bottom of the handle 210 to extend along a height direction. Also, preferably, the racks 211 may be arranged in pairs facing each other, one on each side radially opposite to each other. The first crank 241 and the second crank 242 are arranged to face each rack 211.

When the support portion 230 and the crank 240 are formed by assembling of two parts, there is the effect of reducing the manufacturing difficulty of the applicator 200 and manufacturing cost.

One end of one or more connecting rods 250 is fastened to the crank 240, and the other end thereof is fastened to the needle carrier 260. Specifically, one connecting rod 250 includes a first insertion hole 253 formed in one end of the connecting rod 250, and a first rod protrusion 244 is inserted into the first insertion hole 253. In addition, one connecting rod 250 includes a second insertion hole 254 formed in the other end of the connecting rod 250, and a rod fixing protrusion 261 is inserted into the second insertion hole 254.

One or more connecting rods 250 may include a first connecting rod 251 and a second connecting rod 252. The first connecting rod 251 is fastened to the first crank 241, and the second connecting rod 252 is fastened to the second crank 242.

The needle carrier 260 is subject to a rotation motion and a straight line motion of one or more cranks 240 and may make a straight line motion between the distal position and the proximal position. In this case, the needle carrier 260 is connected to one or more cranks 240 through the connecting rod 250.

The needle carrier 260 is configured to make a straight line motion of the needle 270 from the distal position to the proximal position. Also, when the needle carrier 260 moves from the proximal position to the distal position, the needle 270 moves from the proximal position to the distal position together.

The needle carrier 260 may be formed by assembling two different parts 260a and 260b.

The transmitter holder 280 is coupled to the transmitter 20 and is configured to make a straight line motion of the transmitter 20 from the distal position to the proximal position. Meanwhile, although the transmitter holder 280 makes a reciprocation motion between the distal position and the proximal position, the transmitter 20 moves only in a direction from the distal position to the proximal position.

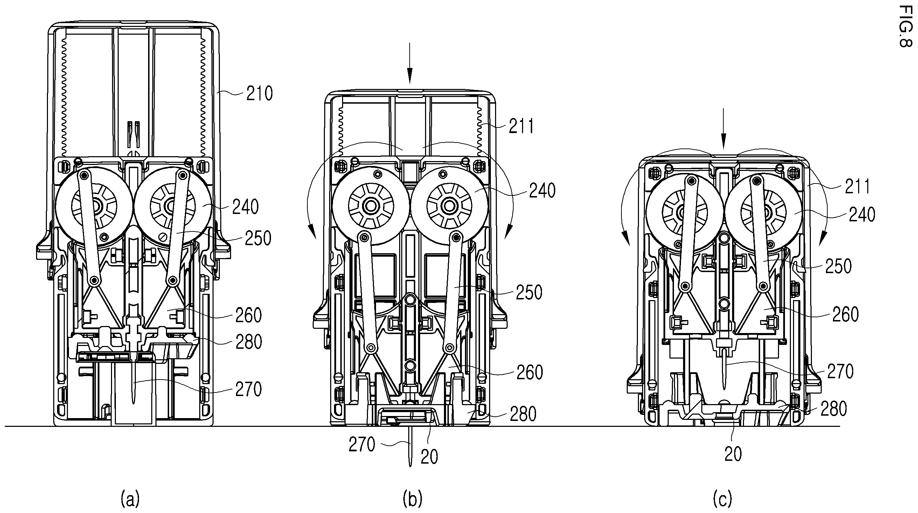

FIG. 8 illustrates an operation of the applicator according to the second embodiment of the present disclosure.

(a) of FIG. 8 illustrates a state before the handle 210 is pressed.

Referring to (a) of FIG. 8, one or more cranks 240 are disposed on a distal end of the rack 211. The support portion 230 is in contact with the skin, and the transmitter 20 and the transmitter holder 280 are in a distal position state separated from the skin. Also, one end of the connecting rod 250 is arranged in the most distal position with respect to one or more cranks 240.

(b) of FIG. 8 illustrates a state in which about the half of the handle 210 is pressed and the needle 270 is inserted into the skin.

Referring to (b) of FIG. 8, the handle 210 is pressed, and one or more cranks 240 rotate. Specifically, the crank 240 disposed on the right rotates clockwise, and the crank 240 disposed on the left rotates counterclockwise, and this is not one rotation but about the half rotates, and the cranks 240 may be arranged in the middle of the rack 211. Thus, the handle 210 has only moved to about a total moving distance along the support portion 230.

In a state (b) of FIG. 8, the transmitter 20 and the transmitter holder 280 are in a proximal state contacting the skin. Also, one end of the connecting rod 250 is disposed in the most proximal position with respect to one or more cranks 240.

(c) of FIG. 8 illustrates that the handle 210 is pressed to the end and the needle 270 is retreated from the skin.

Referring to (c) of FIG. 8, the handle 210 is further pressed, and one or more cranks 240 rotate. Specifically, the crank 240 disposed on the right rotates clockwise, and the crank 240 disposed on the left rotates counterclockwise, and the remaining half of rotation is done, and is disposed on the distal end of the rack 211. Thus, the handle 210 moves to all of a total moving distance along the support portion 230. Also, one end of the connecting rod 250 is disposed again in the distal position with respect to one or more cranks 240.

In a state (c) of FIG. 8, the transmitter 20 is attached to the skin, and the needle carrier 260 is subject to the movement of the connecting rod 250 and moves to the distal position together. Thus, the needle 270 that has been inserted into the skin is drawn out from the skin.

To sum up, while the handle 210 moves from the distal position to the proximal position one way through the procesess of (a) to (c) of FIG. 8, the needle carrier 260 moves from the distal position to the proximal position and again from the proximal position to the distal position reciprocatively. Thus, a reciprocating stroke of inserting and drawing out the needle 270 is possible only with the user's handle pressing action.

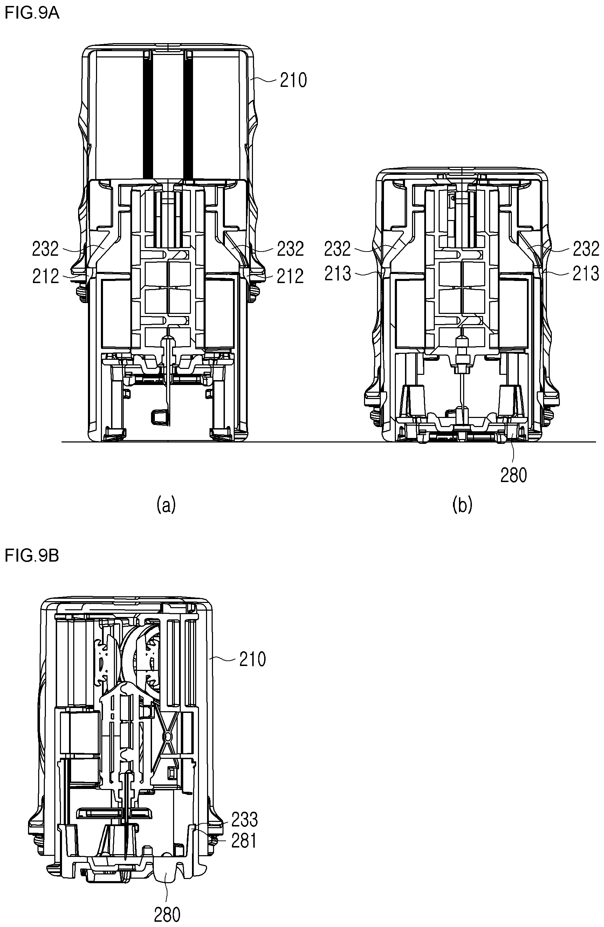

FIG. 9A is a cross-sectional view of FIG. 5B taken along a line II-II'.

Referring to FIG. 9A, the handle 210 includes a first engaging portion 212 and a second engaging portion 213.

The first engaging portion 212 protrudes radially inward from an inner circumferential surface of the handle 210.

The second engaging portion 213 protrudes radially inward from the inner circumferential surface of the handle 210 and is formed in a distal position to the first engaging portion. An inclined surface is formed on the lower surface of the second engaging portion 213, an upper surface may be formed in parallel to a horizontal direction.

The support portion 230 may further include a rib 232. The rib 232 protrudes radially outward from an outer circumferential surface of the support portion 230.

(a) of FIG. 9A represents a state before the handle 210 is pressed.

Referring to (a) of FIG. 9A, the rib 232 of the support portion 230 may be engaged with the first engaging portion 212 of the handle 210, and movement of the rib 232 to the proximal position may be prevented. That is, the rib 232 is engaged with the first engaging portion 212 and thus, the handle may be prevented from being removed from the state (a) of FIG. 9A to the distal position, i.e., upward.

(b) of FIG. 9A represents a state in which the handle 210 is pressed to the end.

Referring to (b) of FIG. 9A, the rib 232 of the support portion 230 is engaged with the second engaging portion 213 of the handle 210, and movement of the rib 232 to the proximal position may be prevented. That is, the rib 232 is engaged with the second engaging portion 213 and thus, the handle may be prevented from being removed from the state (b) of FIG. 9A upward. If the handle 210 is removed from the support portion 230 in the state (b) of FIG. 9A, there is a risk of reuse, but there is the effect of preventing reuse by the second engaging portion 213.

FIG. 9B is a cross-sectional view of FIG. 5B taken along a line III-III'.

In this case, FIG. 9B represents the applicator 200 in a state in which the handle 210 is completely pressed.

Referring to FIG. 9B, the support portion 230 according to the second embodiment of the present disclosure may further include a fixing groove 233. The fixing groove 233 is formed to sink from the inner circumferential surface of the support portion 230 radially outward, and it is preferable that the fixing groove 233 is formed in a position separated from the bottom of the support portion 230 by the height of the transmitter holder 280.

In addition, the transmitter holder 280 includes an anti-separation protrusion 281. The anti-separation protrusion 281 is formed on the upper surface of the transmitter holder 280 and protrudes radially outward. The transmitter holder 280 may be mounted in the fixing groove 233.

In this case, the upper surface of the fixing groove 233 is formed to be parallel to a horizontal plane. Thus, the transmitter holder 280 moves to the proximal position and then does not move to the distal position and may remain in the position.

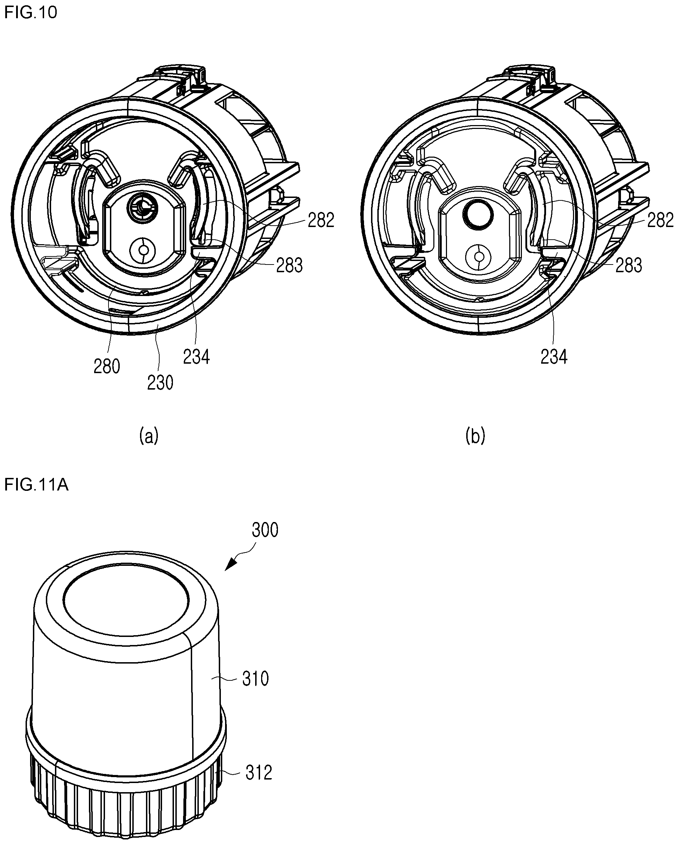

FIG. 10 is an enlarged view of the bottom of the applicator according to the second embodiment of the present disclosure.

Specifically, (a) of FIG. 10 represents a state before the handle 210 is pressed, and (b) of FIG. 10 represents a state in which the handle 210 is pressed to the end.

Referring to FIG. 10, a support portion 230 according to the second embodiment of the present disclosure further includes a hook protrusion 234 that protrudes radially inward from the inner bottom of the support portion 230.

In addition, the transmitter holder 280 may further include a transmitter support portion 230 and a hook 283.

The transmitter support portion 230 is configured to support the transmitter 20 in the distal position and is configured to release a support force provided by the transmitter 20 in the proximal position. To this end, the transmitter support portion 230 is configured to have a cantilever shape, preferably, and the hook 283 may be disposed at a free end of the transmitter support portion 230.

The hook protrusion 234 is formed to be engaged with the hook 283, and the hook 283 is engaged with the hook protrusion 234 so that the free end of the transmitter support portion 230 may be radially outward spread.

### 3. Third embodiment

FIGS. 11A through 11C are perspective views of an applicator according to a third embodiment of the present disclosure.

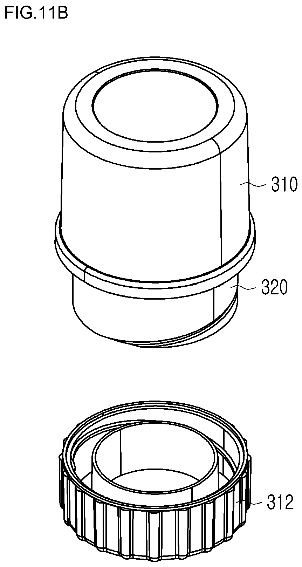

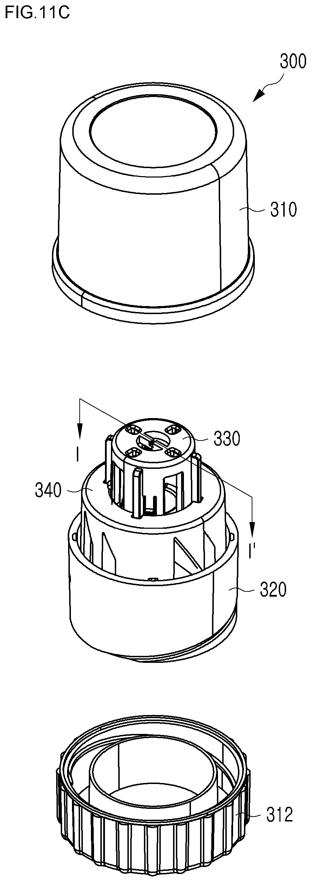

Specifically, FIG. 11A represents a state in which all of an upper cap 310 and a lower cap 312 are mounted, FIG. 11B represents a state in which the lower cap 312 is taken off, and FIG. 11C represents a state in which all of the upper cap 310 and the lower cap 312 are removed.

An applicator 300 according to the third embodiment of the present disclosure includes an upper cap 310 and a lower cap 312 coupled to the upper cap 310 (see FIG. 11A).

When the lower cap 312 is removed from the upper cap 310, a support portion 320 configured in which a bottom end is covered by the upper cap 310 and the lower cap 312, is exposed (see FIG. 11B).

When the upper cap 310 is removed from the support portion 320, the support portion 320, a push cap 330, and a rotation guide 340 are exposed (see FIG. 11C). **In** this case, the rotation guide 340 is arranged to surround a part of the outer circumferential surface of the push cap 330 between the push cap 330 and the support portion 320.

The push cap 330 is coupled to the rotation guide 340 and is configured to make a straight line motion along the rotation guide 340.

The rotation guide 340 is surrounded by the support portion 320.

The upper cap 310, the lower cap 312, the support portion 320, and the push cap 330 according to the third embodiment of the present disclosure have approximately cylindrical shapes, however, the present disclosure is not limited thereto.

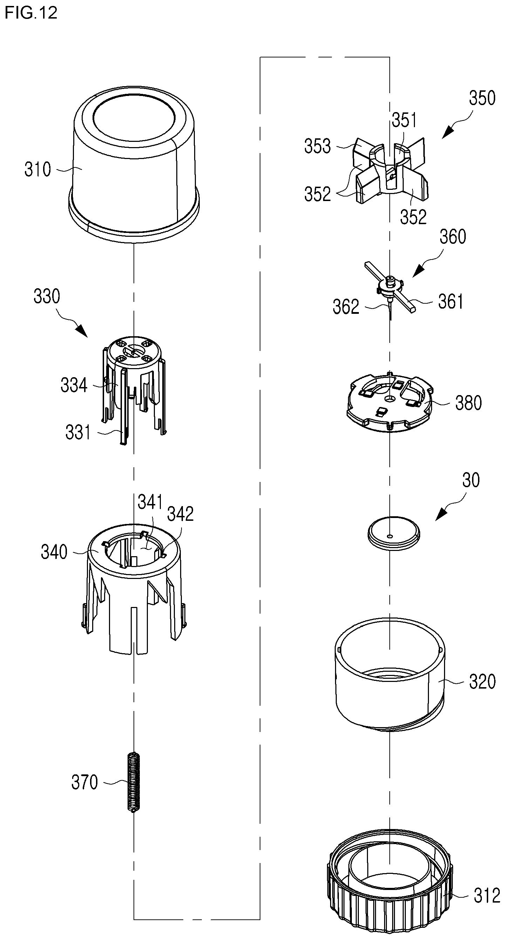

FIG. 12 is an exploded perspective view of an applicator according to the third embodiment of the present disclosure.

The combination relationship between components of an applicator 300 according to the third embodiment will be described with reference to FIG. 12.

The applicator 300 according to the third embodiment includes an upper cap 310, a lower cap 312, a support portion 320, a push cap 330, a rotation guide 340, a rotation block 350, a needle portion 360, a spring 370, and all or part of a transmitter holder 380.

The upper cap 310 and the lower cap 312 are configured to cover at least a part of the support portion 320, and may protect the inside of the applicator 300 from external contaminants or impact.

The support portion 320 is configured so that at least a part of the support portion 320 may be inserted into the upper cap 310 and the lower cap 312, and is configured to surround a part of an outer circumferential surface of the push cap 330. The support portion 320 is in direct contact with the skin during the use of the applicator 300.

The push cap 330 is configured to be pressed by the user, and is configured to make a straight line motion along the height direction of the support portion 320.

The rotation guide 340 is formed to surround a part of the outer circumferential surface of the push cap 330. In this case, the push cap 330 is inserted into an upper hole 341 of the rotation guide 340. Also, the push cap 330 may make a straight line motion along the rotation guide 340. In this case, a push cap guide protrusion 331 formed on the outer circumferential surface of the push cap 330 is inserted into a push cap guide 342 formed on the upper surface of the rotation guide 340 so that the straight line motion of the push cap 330 may be guided.

Meanwhile, it is preferable that a plurality of push cap guide protrusions 331 are arranged along the outer circumferential surface of the push cap 330 at equal intervals, and the present disclosure is not limited thereto, and only one push cap guide protrusion 331 is sufficient.

The push cap 330 may further include a push bar 334. The push bar 334 is in contact with a rotation block 350 in an initial state and may deliver pressure provided by the user to the rotation block 350.

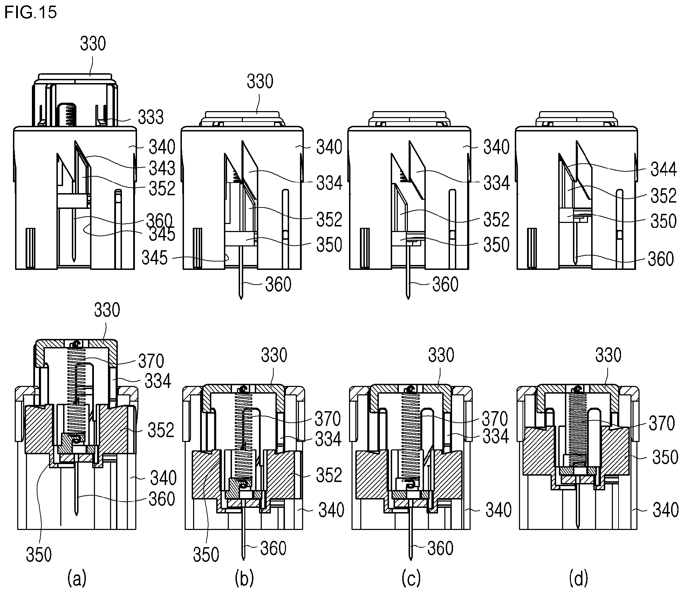

At least a part of the rotation block 350 is arranged inside the rotation guide 340 and may make a straight line motion from the distal position to the proximal position along press-fit of the push cap 330. The rotation block 350 may rotate within the rotation guide 340 by a certain angle. The rotating rotation block 350 makes a straight line motion from the proximal position to the distal position along the rotation guide 340. In this regard, this will be described in detail with reference to FIG. 15.

The rotation block 350 includes a body 351 and a guide wing 352.

The upper surface of the body 351 is open, and preferably, has an approximately cylindrical shape. The body 351 makes a rotation motion and a straight line motion inside the rotation guide 340.

The guide wing 352 protrudes radially outward from an outer circumferential surface of the body 351. The guide wing 352 is inserted into the rotation guide 340 through a main hole (see 345 of FIG. 15) of the rotation guide 340. The upper surface of the guide wing 352 includes an inclined surface 353. The inclination direction of the inclined surface 353 corresponds to the shape of a first seating hole (see 343 of FIG. 15) and a second seating hole (see 344 of FIG. 15). With regard to the motion of the guide wing 352, this will be described in detail with reference to FIG. 15.

Meanwhile, it is preferable that the end of the push bar 334 is formed to have a shape corresponding to the inclined surface 353 of the guide wing 352. That is, the lower surface of the push bar 334 includes an inclined surface, and may be formed in parallel to the inclined surface 353 of the rotation block 350.

The needle portion 360 is coupled to the rotation block 350 and is configured to make a straight line motion of the needle 362 from the distal position to the proximal position. Also, when the needle portion 360 moves from the proximal position to the distal position, the needle 362 moves from the proximal position to the distal position together. The needle portion 360 includes a coupling portion 361 capable of being coupled to the rotation block 350, and a needle 362 that perforates the skin.

The coupling portion 361 includes a circular plate-shaped member, and a rod that protrudes laterally from an outer circumferential surface of the plate-shaped member. In this case, the rod may be formed in plurality. The coupling portion 361 is coupled to the rotation block 350 and may be guided by the rotation guide 340.

One end of the spring 370 is fastened to the push cap 330, and the other end of the spring 370 is fastened to the rotation block 350. In the initial state, the spring 370 in a tensile state is arranged between the push cap 330 and the rotation block 350.

The transmitter holder 380 is coupled to the transmitter 30 and is configured to make a straight line motion of the transmitter 30 from the distal position to the proximal position. Meanwhile, the transmitter holder 380 moves between the distal position and the proximal position reciprocatively, however, the transmitter 30 moves only in a direction from the distal position to the proximal position.

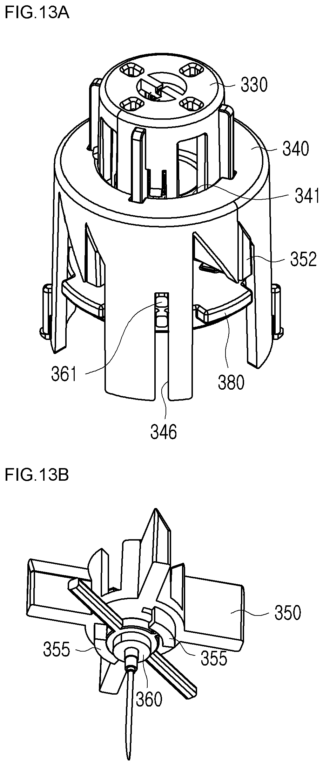

FIGS. 13A and 13B illustrate combination between internal components of the applicator 300 according to the third embodiment of the present disclosure.

Specifically, FIG. 13 illustrates a state in which the push cap 330, the rotation guide 340, the rotation block 350 and the transmitter holder 380 are coupled to each other, i.e., an initial state, and FIG. 13B illustrates a state in which the rotation block 350 and the needle portion 360 are coupled to each other.

Referring to FIG. 13A, the push cap 330 may be inserted into the rotation guide 340 through the upper hole 341 formed in the upper surface of the rotation guide 340.

The guide wing 352 of the rotation block 350 may be inserted into a hole formed in the rotation guide 340.

The needle portion 360 may be coupled to the rotation guide 340 when the coupling portion 361 is inserted into the needle guide 346 formed on the outer circumferential surface of the rotation guide 340. The needle guide 346 may be formed between a plurality of main holes (see 345 of FIG. 15).

The transmitter holder 380 may be inserted into a main hole 345 of the rotation guide 340 and thus may be coupled to the rotation guide 340.

Referring to FIG. 13B, the rotation block 350 may further include a needle mounting portion 355 that protrudes downwardly from an outer bottom. The coupling portion 361 of the needle may be coupled to the needle mounting portion 355.

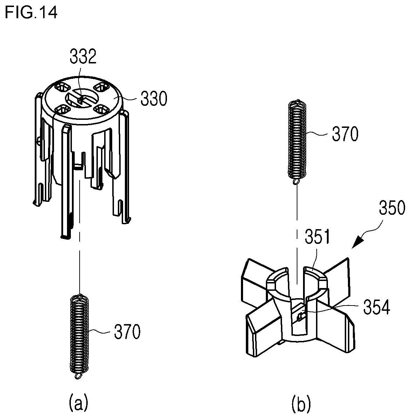

FIG. 14 illustrates a fastening method of a spring according to the third embodiment of the present disclosure. A detailed configuration in which the spring 370 is fastened to the push cap 330 and the rotation block 350, will be described with reference to FIG. 14.

(a) of FIG. 14 illustrates the combination relationship between the push cap 330 and the spring 370, and the inner surface of the push cap 330 and the spring 370 are seen from a viewpoint of a front perspective view.

Referring to (a) of FIG. 14, the push cap 330 includes a first spring holder 332 formed on the upper surface. One end of the spring 370 may be engaged with the first spring holder 332.

(b) of FIG. 14 illustrates the combination relationship between the rotation block 350 and the spring 370, and the rotation block 350 and the spring 370 are seen from a viewpoint of a front perspective view.

Referring to (b) of FIG. 14, the rotation block 350 includes a second spring holder 354 that protrudes inwardly from the inner bottom. The other end of the spring 370 may be engaged with the second spring holder 354. In this case, the second spring holder 354 may protrude from the inner bottom of the body 351.

That is, the push cap 330 and the rotation block 350 are in a state in which they are connected to each other with the spring therebetween, and in this case, the spring 370 is in a tensile state from the initial state, preferably, a state in which it rotates in a tensile direction and in one direction. Thus, the spring 370 may be in a state in which the spring 370 has a potential for compression and rotating in an opposite direction to one direction.

Meanwhile, a distance between the push cap 330 and the rotation block 350 in the initial state may be maintained by the push bar (see 334 of FIG. 12) of the push cap 330.

FIG. 15 illustrates an operation of the applicator according to the third embodiment of the present disclosure.

A first row of FIG. 15 illustrates the front of the applicator 300, and a second row illustrates the cross-section of the applicator 300. In this case, the cross-section is made by cutting a state in which the push cap 330 and the rotation guide 340 of FIG. 11C are coupled to each other, taken along a line I-I'.

Referring to FIG. 15, the push cap 330 may further include an engaging protrusion 333 that protrudes radially outward from the outer circumferential surface. The engaging protrusion 333 may be engaged with an outer upper surface of the rotation guide 340. Thus, the applicator 300 may be prevented from accidental firing due to the user's pressing the push cap 330. Meanwhile, when a certain pressure or more is applied to the engaging protrusion 333, engaging between the engaging protrusion 333 and the push cap 330 may be released. That is, the applicator 300 according to the third embodiment of the present disclosure may naturally induce the user to press the push cap 330 with a certain force or more.

In addition, the rotation guide 340 may further include a first seating hole 343, a second seating hole 344, and a main hole 345.

The first seating hole 343 is formed on the outer circumferential surface of the rotation guide 340 and is formed so that the guide wing 352 may be mounted in the first seating hole 343. To this end, it is preferable that the first seating hole 343 has the same shape as the inclined surface 353 of the guide wing 352.

The second seating hole 344 is formed on the outer circumferential surface of the rotation guide 340 and is formed so that the guide wing 352 may be mounted in the second seating hole 344. In this case, the second seating hole 344 is formed adjacent to the first seating hole 343. It is preferable that an angle between the second seating hole 344 and the first seating hole 343 is formed smaller than an angle kat which the spring 370 is rotated in the initial state.

The main hole 345 is formed on the outer circumferential surface of the rotation guide 340, and is formed below the first seating hole 343 and the second seating hole 344. The first seating hole 343 and the second seating hole 344 have a shape in which they merges into the main hole 345 from the bottom. In other words, the first seating hole 343 and the second seating hole 344 are diverged from the main hole 345.

Hereinafter, an operating method of the applicator 300 as described above will be described in detail.

(a) of FIG. 15 illustrates a state before the push cap 330 is pressed.

Referring to the front view of (a) of FIG. 15, the push cap 330 and the rotation block 350 are arranged in the distal position. In this case, the guide wing 352 of the rotation block 350 is mounted in the first seating hole 343. Referring to the cross-sectional view of (a) of FIG. 15, since, in the initial state, the push bar 334 presses the inclined surface 353 of the guide wing 352, the push cap 330 and the rotation block 350 are spaced apart from each other by a certain distance. Thus, the spring 370 is in a tensile state and rotating state.

(b) of FIG. 15 illustrates a state in which the push cap 330 is pressed, and illustrates a state in which the rotation block 350 moves from the distal position to the proximal position. In this case, the guide wing 352 of the rotation block 350 moves to the proximal position while being arranged in parallel to the first seating hole 343. Referring to the cross-sectional view of (b) of FIG. 15, the push bar 334 still presses the inclined surface 353 of the guide wing 352, and the push cap 330 and the rotation block 350 are spaced apart from each other by a certain distance. Also, the spring 370 is still in a tensile and rotating state.

(c) of FIG. 15 illustrates a state in which the rotation block 350 rotates in an opposite direction to one direction from the proximal position when the push cap 330 is pressed. In this case, the guide wing 352 of the rotation block 350 rotates in an opposite direction to one direction in a proximal state. This means that the spring 370 is rotated by a potential that the spring 370 possesses until the process (b). Meanwhile, due to rotation of the spring 370, the rotation block 350 fastened to the spring 370 is rotated together.

Referring to all of the front view and the cross-sectional view of (c) of FIG. 15, due to rotation of the rotation block 350, the inclined surface 353 of the guide wing 352 does not face the push bar 334 any more. Thus, a configuration of suppressing the movement of the guide wing 352 to the distal position is not present and thus, the spring 370 may be in a compressible state.

(d) of FIG. 15 illustrates a state in which the rotation block 350 moves from the proximal position to the distal position when the push cap 330 is pressed. When the spring 370 is pressed, the rotation block 350 is mounted in the second seating hole 344. As described above, the push bar 334 and the guide wing 352 are located in crossed positions, the rotation block 350 may move to the distal position.

Meanwhile, although, in the present disclosure, operations (a) to (d) of FIG. 15 are shown as being performed at regular temporal intervals, operations (b) to (d) are performed by the moment the user presses the push cap 330. Thus, since the operations are performed faster than the average user's recognition speed, the user may use the applicator 300 simply and without much pain.

In addition, when the applicator 300 according to the third embodiment of the present application is used, the needle may be inserted into and drawn out from the skin only with the user's operation of pressing the push cap 330 so that this is intuitive and simple and pain may be minimized.

### 4. Fourth embodiment

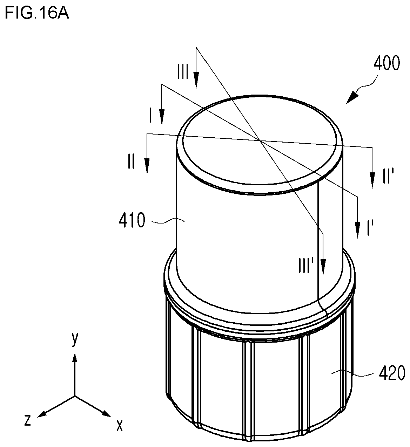

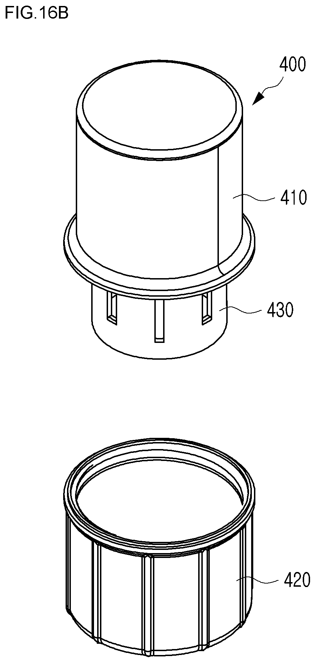

FIGS. 16A and 16B are perspective views of an applicator according to a fourth embodiment of the present disclosure.

Specifically, FIG. 16A illustrates a state in which the cap 420 is mounted, and FIG. 16B illustrates a state in which the cap 420 is removed.

The applicator 400 according to the fourth embodiment of the present disclosure includes a handle 410 and a cap 420 that is detachable from the handle 410 (see FIG. 16A).

When the cap 420 according to the fourth embodiment of the present disclosure is removed from the handle 410, a part of a cylinder 430 is exposed (see FIG. 16B). In this case, at least a part of a piston 440 is inserted into the handle 410.

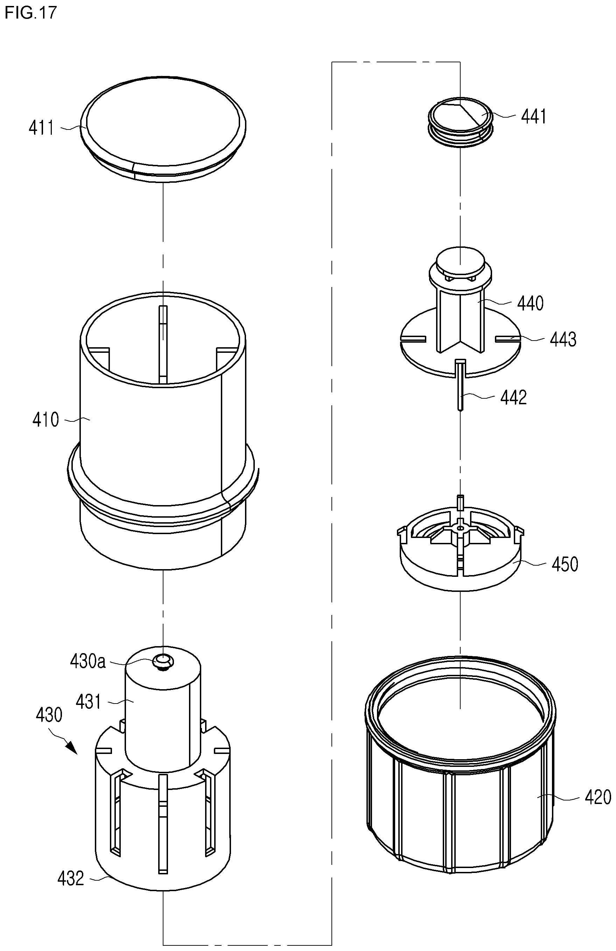

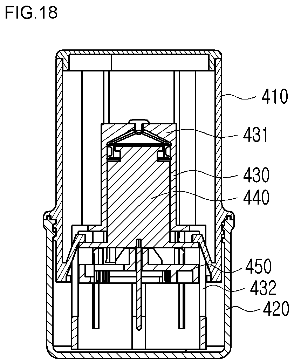

FIG. 17 is an exploded perspective view of the applicator according to the fourth embodiment of the present disclosure. FIG. 18 is a cross-sectional view of FIG. 16A taken along a line I-I'. The combination relationship between components of the applicator 400 according to the fourth embodiment will be described with reference to FIGS. 17 and 18. Meanwhile, FIG. 18 illustrates an appearance before use of the applicator 400 after the applicator is manufactured, and here, when the cap 420 is removed, an initial state for use is established.

The applicator 400 according to the fourth embodiment includes a handle 410, a cap 420, a cylinder 430, a piston 440, and all or part of a transmitter holder 450.

The handle 410 is configured to be grasped by the user, and preferably, has an approximately cylindrical shape. The upper surface of the handle 410 is covered by a handle cover 411. The handle cover 411 is coupled to the handle 410 so that an accommodation space is formed inside the handle 410, and the assembly process is easier because only the handle cover 411 needs to be assembled in the final step after the internal assembly of the applicator 400. However, the present disclosure is not limited thereto, and the handle 410 and the handle cover 411 may be formed as an integral part according to the designer's selection.

The cap 420 is configured to be mounted on or removed from the handle 410, and may protect the inside of the applicator 400 from external contaminants or impact. It is preferable that the cap 420 has an approximately cylindrical shape.

The cylinder 430 is configured so that at least a part of the cylinder 430 is inserted into the handle 410. The cylinder 430 includes a first body 431 and a second body 432 disposed below the first body 431. Preferably, the diameter of the first body 431 is formed smaller than the diameter of the second body 432.

The cylinder 430 is in direct contact with the user's skin when the applicator 400 is used.

The piston 440 is configured so that at least a part of the piston 440 is inserted into the cylinder 430. The outer diameter of the piston 440 is configured to be the same as or slightly smaller than the inner diameter of the first body 431. Thus, the piston 440 is pressed into the first body 431, and the space between the first body 431 and the piston 440 has limited fluid communication with the outside air.

A rubber packing 441 is coupled to the end of the piston 440. The inside of the first body 431 into which the piston 440 is inserted, may be sealed by the rubber packing 441 and the packing 430a. Meanwhile, the rubber packing 441 may be double-injected and formed into the piston 440 in a manufacturing operation, however, the present disclosure is not limited thereto.

A flange 443 is formed on one surface of the piston 440. The diameter of the flange 443 is greater than the inner diameter of the first body 431 and is smaller than the inner diameter of the second body 432.

In addition, a needle 442 is attached to one surface of the flange. The needle 442 is configured to perforate and to be inserted into the user's skin and may perforate the transmitter holder 450 and a transmitter (not shown).

The transmitter holder 450 is coupled to the transmitter (not shown) and is configured to make a straight line motion of the transmitter from the distal position to the proximal position. Meanwhile, the transmitter holder 450 and the transmitter may move only in a direction from the distal position to the proximal position.

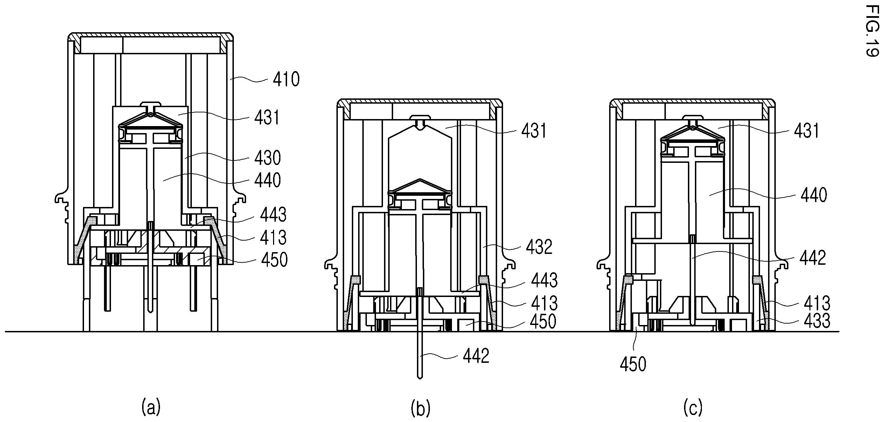

The transmitter holder 450 is configured to make a motion by pressing of the piston 440 from the distal position to the proximal position, and in this regard, this will be described in detail with reference to FIG. 19.

FIG. 19 illustrates an operation of the applicator according to the fourth embodiment of the present disclosure. Specifically, FIG. 19 illustrates a process in which the applicator 400 operates, as a cross-sectional view of FIG. 16A taken along a line I-I'.

Referring to FIG. 19, the handle 410 includes a cantilever-shaped hook 413 having one end that is a free end. The hook 413 is disposed on an inner circumferential surface of the handle 410 and is configured to be hung and fitted into the flange 443 of the piston 440. In this case, it is preferable that a fixing end of the hook 413 is disposed below the free end. Thus, the hook 413 may press the flange 443 in a movement direction while the piston 440 moves from the distal position to the proximal position.

Meanwhile, the cylinder 430 includes a hook resistance part 433. The hook resistance part 433 protrudes radially inward from the inner circumferential surface of the cylinder 430. The hook resistance part 433 is configured to release hung and fit of the hook 413, and hereinafter, an operating method of the hook 413 and the hook resistance part 433 will be described.

(a) of FIG. 19 illustrates a state before the handle 410 is pressed.

Referring to (a) of FIG. 19, the handle 410 is spaced apart from the user's skin. Also, the piston 440 is pressed into the first body 431 of the cylinder 430. Since a very small amount of air is present between the piston 440 and the first body 431, it is preferable to have a sub-vacuum state.

The hook 413 of the handle 410 is hung and fitted into the flange 443.

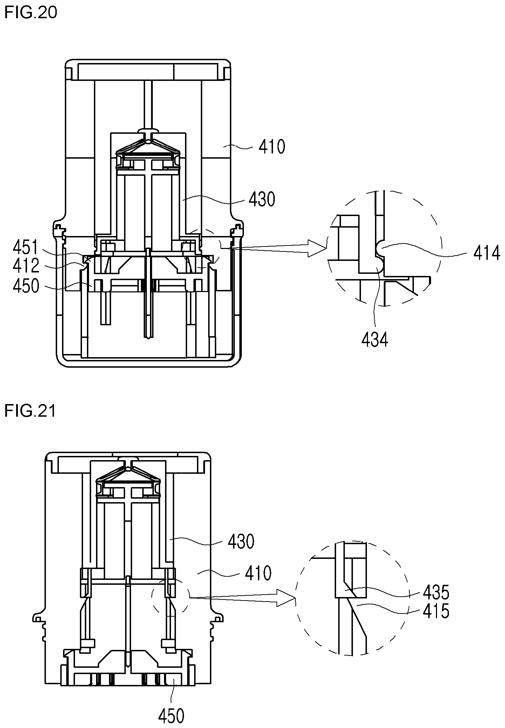

The transmitter holder 450 is supported by the handle 410 and is disposed at the distal position. A configuration in which the transmitter holder 450 is supported by the handle 410, will be described in detail with reference to FIG. 20.

(b) of FIG. 19 illustrates a state in which the handle 410 is pressed and the needle 442 is inserted into the skin.

Referring to (b) of FIG. 19, when the user presses the handle 410, the piston 440 moves from the distal position to the proximal position. Meanwhile, when the handle 410 moves to the proximal position, the hook 413 is in contact with the hook resistance part 433, and when the handle 410 further moves to the more proximal position, the hook 413 moves outwardly. In other words, as the hook 413 is disengaged by the hook resistance part 433, the combination between the hook 413 and the flange 443 may be released. Thus, the transmitter holder 450 may not be constrained by the movement of the piston 440.

Meanwhile, as the piston 440 moves from the distal position to the proximal position, the needle 442 is inserted into the user's skin.

In the state (b) of FIG. 19, as the volume between the piston 440 and the first body 431 increases, the internal pressure becomes very small. Thus, the he piston is then forced back toward the distal position. In this case, since the hook 413 of the handle 410 does not constrain the flange 443 of the piston 440 any more, the piston 440 is in a state in which it returns to the distal position again.

Meanwhile, since the flange 443 of the piston 440 is not fastened to the hook 413 any more, the piston 440 is not constrained by the pressing of the handle 410. Thus, even when the handle 410 is pressed again, the state of the piston 440 will not be changed in a state in which it is inserted into the distal end of the cylinder 430. That is, the reuse of the applicator 400 becomes impossible.

Since the needle is inserted into the user's skin once, it may be exposed to infection with various diseases when it is reused. Thus, when the reuse of the applicator is not necessary, the needle may also be used. In the applicator 400 according to the fourth embodiment of the present disclosure, reuse may be structurally prevented.

(c) of FIG. 19 illustrates a state in which the needle 442 is drawn out from the skin.

Referring to (c) of FIG. 19, the handle 410 is located in the proximal position, and the piston 440 moves from the proximal position to the distal position. By the movement of the piston 440, the needle 442 inserted into the user's skin may be drawn out. The piston 440 is inserted into the first body 431 again.

The transmitter holder 450 is maintained in the proximal position, i.e., a state close to the skin.

FIG. 20 is a cross-sectional view and a partially-enlarged view of FIG. 16A taken along a line II-II'.

FIG. 20 illustrates an initial state, and describes a configuration for preventing firing in the initial state, and the combination relationship between the transmitter holder 450 and the handle 410 will be described with reference to FIG. 20.

Referring to FIG. 20, the handle 410 includes a first forward-preventing protrusion 414. The first forward-preventing protrusion 414 protrudes radially inward from the inner circumferential surface of the handle 410.

In addition, the cylinder 430 includes a second forward-preventing protrusion 434. The second forward-preventing protrusion 434 protrudes radially outward from the outer circumferential surface of the cylinder 430.

In the initial state, the first forward-preventing protrusion 414 and the second forward-preventing protrusion 434 face each other. Specifically, the lower surface of the first forward-preventing protrusion 434 and the upper surface of the second forward-preventing protrusion 434 may face each other.

Meanwhile, the lower surface of the first forward-preventing protrusion 414 and the upper surface of the second forward-preventing protrusion 434 are configured to be inclined from the distal position to the proximal position. Thus, the handle 410 may not be pressed by force below a certain size, and the handle 410 may move from the distal position to the proximal position only by applying force above a certain size. That is, in the initial state, the handle 410 is pressed by unintentional impact so that the applicator 400 can be prevented from firing, and when the user intends to use it, it is possible to induce a pressure greater than the level at which it can be pressed all the way to the end. Thus, a phenomenon that the needle is not inserted due to stopping in the middle when the handle 410 is pressed, can be prevented.

The handle 410 may further include a first fixing protrusion 412. The first fixing protrusion 412 protrudes radially inward from the inner circumferential surface of the handle 410.

In addition, the transmitter holder 450 includes a second fixing protrusion 451. The second fixing protrusion 451 protrudes radially outward from the outer circumferential surface of the transmitter holder 450.

In the initial state, the first fixing protrusion 412 and the second fixing protrusion 451 are engaged with each other, and specifically, the second fixing protrusion 451 is disposed on the first fixing protrusion 412. Thus, the transmitter holder 450 may be supported by the handle 410.

Meanwhile, it is preferable that the upper surface of the first fixing protrusion 412 and the lower surface of the second fixing protrusion 451 are formed in parallel to the horizontal plane. Thus, in the initial state, the transmitter holder 450 may be stably fixed without being deviated from the distal position.

FIG. 21 is a cross-sectional view and a partially-enlarged view of FIG. 16A taken along a line III-III'.

FIG. 21 illustrates a state after the needle returns after firing, and a configuration for preventing reuse of the applicator 400 will be described with reference to FIG. 21.

Referring to FIG. 21, the handle 410 may further include a first retreat-preventing protrusion 415. The first retreat-preventing protrusion 415 protrudes radially outward from the inner circumferential surface of the handle 410.

In addition, the cylinder 430 may further include a second retreat-preventing protrusion 435. The second retreat-preventing protrusion 435 protrudes radially outward from the outer circumferential surface of the cylinder 430.

In the finished state, the first retreat-preventing protrusion 415 and the second retreat-preventing protrusion 435 face each other, and specifically, the second retreat-preventing protrusion 435 is disposed on the first retreat-preventing protrusion 415. Thus, the handle 410 can be prevented from retreating to the distal position again from moving to the proximal position.

Meanwhile, it is preferable that the upper surface of the first retreat-preventing protrusion 415 and the lower surface of the second retreat-preventing protrusion 435 are formed in parallel to the horizontal plane. Thus, in the finished state, the handle 410 and the piston 440 may not deviate from the proximal position. That is, since the piston 440 may not move from the proximal position to the distal position any more, the needle 442 can be prevented from protruding toward the outside of the applicator 400 again. Thus, there is the effect of preventing the user from getting stabbed or being injured by the needle 442 contaminated after use.

### 5. 5^{th}-1 embodiment

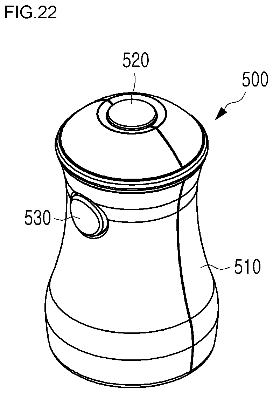

FIG. 22 is a perspective view of an applicator according to a 5^{th}-1 embodiment of the present disclosure.

Referring to FIG. 22, an applicator 500 according to the 5^{th}-1 embodiment of the present disclosure includes a housing 510.

The housing 510 is configured to be grasped by a user, and preferably, has a hollow column shape. Also, the middle of the housing 510 may have a concave shape so that the user may grasp the entire housing 510. Meanwhile, the applicator 500 according to the 5^{th}-1 embodiment of the present disclosure operates in an automatic firing method, by which the applicator 500 is automatically fired when pressing a button rather than the user's direct pressing, unlike in the first through fourth embodiments described above.

To this end, the applicator 500 further includes a first button 520 and a second button 530.

The first button 520 is disposed on the upper surface of the housing 510 and is configured to be pressed downward.

The second button 530 is disposed on the outer circumferential surface of the housing 510 and is configured to be pressed toward the inside of the housing 510.

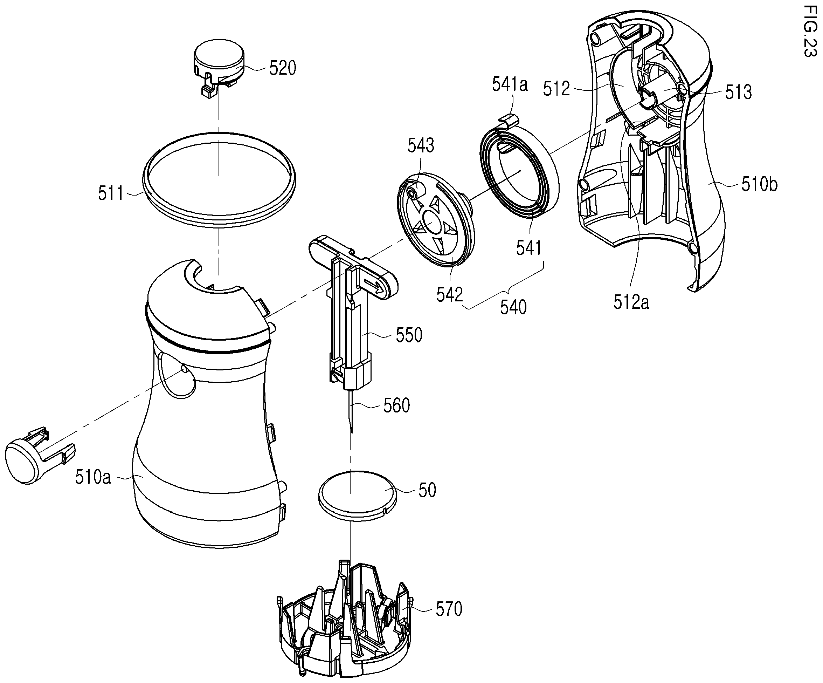

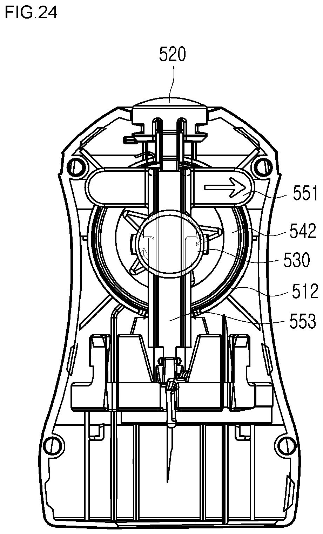

FIG. 23 is an exploded perspective view of the applicator according to the 5^{th}-1 embodiment of the present disclosure. FIG. 24 illustrates the inside of the applicator according to the 5^{th}-1 embodiment of the present disclosure.

The combination relationship between components of the applicator 500 according to the 5^{th}-1 embodiment of the present disclosure will be described with reference to FIGS. 23 and 24.

The applicator 500 according to the fifth embodiment includes a housing 510, a first button 520, a second button 530, a driving portion 540, a needle carrier 550, and all or part of a transmitter holder 570.

The bottom of the housing 510 is in contact with the user's skin and is configured to insert a sensor mounted on the transmitter 50.

Preferably, the housing 510 may be manufactured by injection molding. In this case, a first configuration 510a and a second configuration 510b may be individually manufactured, and the first configuration 510a and the second configuration 510b may be assembled to each other so that the housing 510 may be formed.

The housing 510 may include a housing fixing portion 511, a drum 512, and a wheel fixing portion 513.

The housing fixing portion 511 is configured to be combined with the first configuration 510a and the second configuration 510b. Specifically, the housing fixing portion 511 is configured to be fastened to the outer circumferential surface of at least a part, preferably, an upper end of the first configuration 510a and the second configuration 510b. Thus, the assembly of the first configuration 510a and the second configuration 510b may be stably fixed. When external impact is applied to the applicator 500, although there is a risk that the assembled housing 510 may be disassembled, the housing fixing portion 511 maintains the combination of the first configuration 510a and the second configuration 510b firmly so that the disassembling of the applicator 500 can be prevented.

The drum 512 is formed to correspond to the entire shape of a spring so that the spring 541 may be disposed inside the drum 512. As will be described below, the spring 541 according to the fifth embodiment of the present disclosure has a wound roll spring shape and thus, preferably, the drum 512 has an approximately cylindrical shape.

A spring fixing groove 512a configured so that an outside end 541a of the spring may be mounted within the spring fixing groove 512a, may be formed on the outer circumferential surface of the drum 512. The spring fixing groove 512a may have a shape in which at least a part of the outer circumferential surface of the drum 512 is cut.

A wheel fixing portion 513 is disposed in the center of the drum 512 and protrudes radially inward from the inner circumferential surface of the second configuration 510b. The center of a wheel 542 passes through the wheel fixing portion 513 so that the wheel 542 may be stably held in the wheel fixing portion 513. Also, it is preferable that the wheel fixing portion 513 has a column shape with a smooth outer circumferential surface so that the wheel 542 may rotate around the wheel fixing portion 513.

The first button 520 is configured to be inserted into the housing 510, and is configured to be pressed in a direction from the distal position to the proximal position.

The second button 530 is configured to be inserted into the housing 510, and is configured to be pressed in a horizontal direction.

The applicator 500 may be fired only when both the first button 520 and the second button 530 are pressed. In this case, the order in which the first button 520 and the second button 530 are pressed, does not matter. That is, the first button 520 and the second button 530 may function as both a safety unlock button and a pressing button.

The driving portion 540 is configured to make a straight line motion of the needle carrier 550 and the needle 560 from the distal position to the proximal position in response to the pressing of the first button 520 and the second button 530. To this end, the driving portion 540 may include a spring 541, a wheel 542, and a guide protrusion 543.

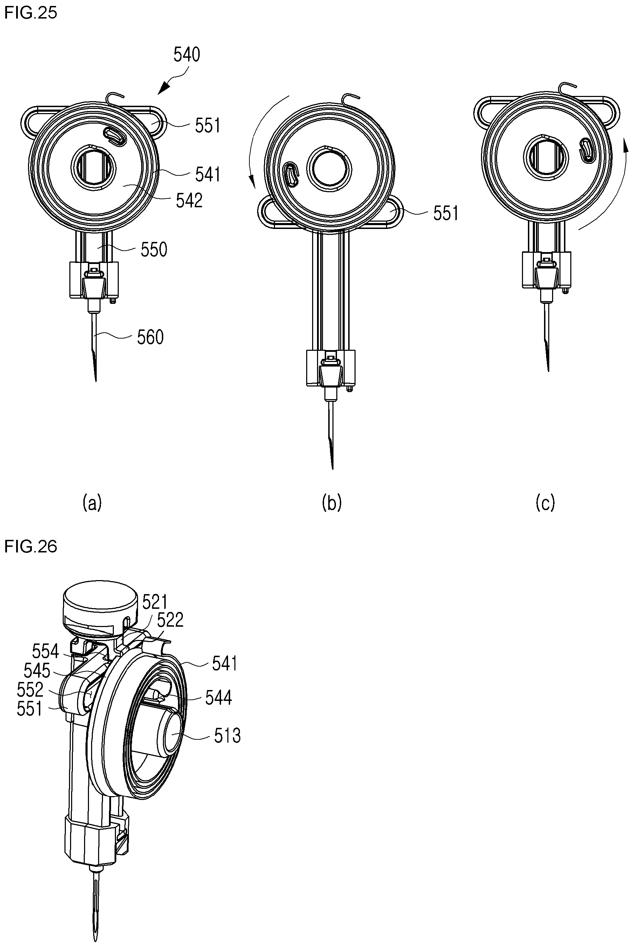

One end of the spring 541 is fastened to the wheel 542, and the other end of the spring 541 is fastened to one surface of the drum 512. Here, one end means an end disposed inside the spring 541, and the other end means an end disposed outside the spring 541. One end may be hung and fixed in a protrusion (see 544 of FIG. 26) formed in the wheel 542, and the other end may be and fixed in a fixing protrusion (not shown) separately provided on one surface of the drum.

The spring 541 is a power source for providing power so that the straight line motion of the needle carrier 550 is possible. In this case, preferably, the spring 541 is a wound roll spring. The spring 541 is compressed and assembled in the initial state. By pressing of the first button 520 and the second button 530, a fastening portion for fixing compression of the spring 541 is released, and the spring 541 is tensioned to return its original state. Thus, the inside end of the spring 541 rotates in an opposite direction to the compressed direction. That is, it may be based on the principle of a kind of scotch yoke or double slider crank mechanism, whereby the rotational motion of the spring 541 is converted into the straight line motion of the needle carrier 550 and the needle 560. In this regard, this will be described in detail with reference to FIG. 25.

The wheel 542 is coupled to the drum 512 and is configured so that an accommodation space may be formed inside the wheel 542. The spring 541 may be disposed in the accommodation space formed between the wheel 542 and the drum 512. Additionally, the wheel 542 is fastened to the spring 541 and is configured to rotate together by rotation of the spring 541.

The guide protrusion 543 protrudes from one surface of the wheel 542 toward the inside of the applicator 500.

The needle carrier 550 is configured to make a straight line motion inside the housing 510. In this case, the straight line motion of the needle carrier 550 may be induced by the rotation motion of the driving portion 540. To this end, the needle carrier 550 may include a lateral guide 551 and a longitudinal guide 553, and the needle carrier 550 may have an overall shape of the English letter T (see FIG. 24).

The guide protrusion 543 of the driving portion 540 may be inserted into the lateral guide 551. When the wheel 542 rotates, the guide protrusion 543 may rotate, and thus, the guide protrusion 543 may move left and right along the lateral guide 551. Thus, the needle carrier 550 makes a straight line motion between the distal position and the proximal position.

The lateral guide 551 is configured to be fastened to the first button 520, and is movable only when fastening with the first button 520 is released. In this regard, this will be described in detail with reference to FIG. 26.

The longitudinal guide 553 extends from one surface of the lateral guide 551 in a direction from the distal position to the proximal position. The longitudinal guide 553 is combined with the needle 560 to fix the needle 560.

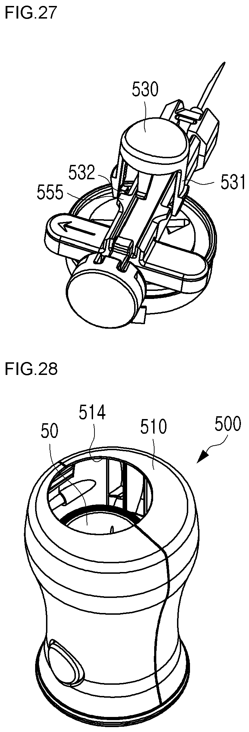

The longitudinal guide 553 is configured to be fastened to the second button 530, and is movable only when fastening with the second button 530 is released. In this regard, this will be described in detail with reference to FIG. 27.

The needle 560 is coupled to the needle carrier 550, is constrained by the straight line motion of the needle carrier 550 and is configured to make a straight line motion together. The needle 560 is configured to perforate the user's skin.

The transmitter holder 570 is coupled to the transmitter 50 and is configured to make a straight line motion of the transmitter 50 from the distal position to the proximal position. The transmitter holder 570 is pressed from the distal position to the proximal position by the longitudinal guide 553. Meanwhile, the transmitter holder 570 moves only in a direction from the distal position to the proximal position.

Meanwhile, since the housing 510 according to the present disclosure is divided into two parts and is manufactured, in the manufacturing process of the applicator 500, the spring 541, the wheel 542, the needle carrier 550, the first configuration 510a, and the second button 530 in this order may be sequentially stacked on the second configuration 510b and may be assembled. Thus, the assembling process of the applicator 500 is simple, and manufacturing cost can be reduced.

FIG. 25 illustrates the operating principle of the applicator according to the 5^{th}-1 embodiment of the present disclosure.

(a) of FIG. 25 illustrates a state in which any one more of the first button 520 and the second button 530 are not pressed, i.e., an initial state.

Referring to (a) of FIG. 25, the guide protrusion 543 is disposed in the most distal position, and thus, the needle carrier 550 may also be disposed in the distal position. Meanwhile, the spring 541 in this case may be in a tensile state.

Meanwhile, according to (a) of FIG. 25, since any one or more of the first button 520 and the second button 530 are not pressed, the motion of the needle carrier 550 to the proximal position is limited by the first button 520 or the second button 530. Thus, the motions of the guide protrusion 543, the wheel 542, and the spring 541 directly/indirectly coupled to the needle carrier 550 are limited.

(b) of FIG. 25 illustrates a state in which both the first button 520 and the second button 530 are pressed and the needle 560 is inserted into the skin.

Referring to (b) of FIG. 25, while the first button 520 and the second button 530 are pressed, fastening between the first button 520 and the lateral guide 551 and fastening between the second button 530 and the longitudinal guide 553 are released. Thus, the motion of the needle carrier 550 from the distal position to the proximal position is not limited any more. Thus, the motions of the guide protrusion 543, the wheel 542, and the spring 541 directly/indirectly coupled to the needle carrier 550 are possible.

The spring 541 rotates in one direction (illustrated counterclockwise in FIG. 25 of the present disclosure, however, the opposite direction is also acceptable). However, to allow rotation in the opposite direction, the direction of the hook formed at the other end of the spring 541 needs to be opposite.

The guide protrusion 543 is disposed in the most proximal position, and thus, the needle carrier 550 is also disposed in the proximal position. Meanwhile, the spring 541 may be in a state in which the tension of about the half of the spring 541 is released.

While the needle carrier 550 moves to the proximal position, the needle 560 may move to the proximal position and may be inserted into the user's skin.

(c) of FIG. 25 illustrates a state in which the needle 560 is drawn out from the user's skin.

Referring to (c) of FIG. 25, the spring 541 further rotates in one direction.

Therefore, the guide protrusion 543 is disposed at the distal position again and thus, the needle carrier 550 is also disposed at the distal position. Meanwhile, the tension of the spring 541 is fully released, and a driving force is not generated by the spring 541.

While the needle carrier 550 moves to the distal position again, the needle 560 may move to the distal position and may be drawn out from the user's skin.

FIGS. 26 and 27 illustrate a configuration for preventing firing of the applicator according to the 5^{th}-1 embodiment of the present disclosure. Specifically, FIG. 26 illustrates fastening between the first button 520 and the longitudinal guide 553.

Referring to FIG. 26, the first button 520 of the present disclosure may further include an extension portion 521 and an engagement protrusion 522.

The extension portion 521 protrudes downwardly from the lower surface of the first button 520 and is configured to be inserted into one surface of the longitudinal guide 553. Preferably, the extension portion 521 may be formed as a pair that faces each other.

The engagement protrusion 522 protrudes from one surface of the extension portion 521. In this case, it is preferable that a protruding direction is a direction toward a radial outside of the applicator 500 when the applicator 500 is assembled. In addition, it is preferable that the engagement protrusion 522 is formed to be spaced apart from the lower surface of the first button 520. Through a space between the lower surface of the first button 520 and the engagement protrusion 522, a firing-preventing portion 545 to be described below may be arranged.

The engagement protrusion 522 may prevent rotation of the wheel 542 while facing the driving portion 540, especially, part of the wheel 542.

The driving portion 540 may further include a spring fixing protrusion 544 and the firing preventing portion 545.

The spring fixing protrusion 544 is formed on one surface of the wheel 542. In this case, one surface of the wheel 542 means a surface on which the spring 541 is disposed. One end of the spring 541 is fastened to the spring fixing protrusion 544, and thus, the relative position of the spring 541 and the wheel 542 may be fixed. Meanwhile, here, an end of the spring 541 means an end disposed inside the spring 541. While the spring 542 is compressed to return to it's original state from the tensile state, the inside end of the spring 541 is rotated. The spring fixing protrusion 544 and the spring 541 are fastened to each other so that the wheel 542 may rotate in response to the compression process of the spring 541.

The firing preventing portion 545 is formed on the other surface of the wheel 542. In this case, the other surface of the wheel 542 is a surface formed in an opposite direction to one surface, and means a surface facing the needle carrier 550. The firing preventing portion 545 may protrude from the other surface of the wheel 542 while drawing an arc shape along the circumference of the other surface of the wheel 542. Here, it is preferable that a protruding direction is a direction toward a radial outside of the applicator 500 when the applicator 500 is assembled. In other words, the engagement protrusion 522 and the firing preventing portion 545 are formed in a position where they protrude in opposite directions and may be engaged with each other.

The needle carrier 550 may further include a first button combining portion 554.

The first button combining portion 554 is a kind of groove or hole formed in the upper surface of the lateral guide 551, and at least a part of the extension portion 521 of the first button 520 is inserted into the groove. In an initial state, the extension portion 521 of the first button 520 may be a state in which it is not inserted into the first button combining portion 554. However, it is preferable that, when the first button 520 is pressed, the extension portion 521 is inserted into the first button combining portion 554. Thus, downward movement of the first button may be secured.

To sum up, in the initial state, the engagement protrusion 522 of the first button 520 is engaged with the firing preventing portion 545 of the wheel 543. In this case, when the first button 520 is pressed, the extension portion 521 is inserted into the first button combining portion 554, and the first button 520 moves to the proximal position. In this case, the engagement protrusion 522 moves to the proximal position so that engagement of the firing preventing portion 545 and the engagement protrusion 522 is released. Thus, the wheel 542 may be in a rotatable state.

In addition, the needle carrier 550 may further include an insertion groove 552. The insertion groove 552 is a groove recessed from one surface of the longitudinal guide 553. In this case, one surface of the longitudinal guide 553 means a surface facing the wheel 542. The guide protrusion 543 of the wheel 542 may be inserted into the insertion groove 552, and the guide protrusion 543 may move left and right from the insertion groove 552.

FIG. 27 illustrates fastening between the second button 530 and the lateral guide 551.

Referring to FIG. 27, the second button 530 may further include an extension portion 531 and a hook 532.

The extension portion 531 protrudes from the lower surface of the second button 530 and is configured to be inserted into one surface of the lateral guide 551. In this case, it is preferable that a protruding direction is a direction toward a radial outside of the applicator 500 when the applicator 500 is assembled. Preferably, the extension portion 531 may be formed as a pair facing each other.

The hook 532 protrudes from the inside surface of the extension portion 531 toward a radial inside of the second button 530. It is preferable that the hook 532 is spaced apart from the lower surface of the second button 530. Through a space between the lower surface of the second button 530 and the hook 532, a hook protrusion 555 to be described below may be disposed.

The hook 532 may face the needle carrier 550, especially, the longitudinal guide 553, to prevent the straight line motion of the needle carrier 550.

To this end, the needle carrier 550 may further include a hook protrusion 555. The hook protrusion 555 protrudes from one surface of the longitudinal guide 553 toward the radial outside of the second button 530. That is, the hook 532 and the hook protrusion 555 may protrude in opposite directions, and thus may be formed in a position where they are engaged with each other.

To sum up, in the initial state, the hook 532 of the second button 530 is hung in the hook protrusion 555 of the needle carrier 550. In this case, when the second button 530 is pressed, the second button 530 moves to the radial inside of the applicator 500. Then, the hook 532 moves together, and engagement of the hook protrusion 555 and the hook 532 is released. Thus, the needle carrier 550 may be in a state where it may make a straight line motion.

Referring to FIGS. 26 and 27, in the applicator 500 according to the 5^{th}-1 embodiment, for firing, both the first button 520 and the second button 530 need to be pressed.

That is, when the first button 520 and the second button 530 are first pressed, they function as safety unlock buttons, and when the first button 520 and the second button 530 are continuously pressed, they function as firing buttons.

Meanwhile, pressing of the first button 520 and pressing of the second button 530 is an independent mechanism and thus, the first button 520 and the second button 530 may be pressed regardless of the order.

FIG. 28 is a bottom perspective view of the applicator according to the 5^{th}-1 embodiment of the present disclosure.

Referring to FIG. 28, in the applicator 500, an opening 514 is formed on the bottom. In this case, it is preferable that the diameter of the opening 514 is formed to be the same as or slightly greater than the maximum diameter of the transmitter 50. Thus, a phenomenon that contaminants enter the applicator 500, can be prevented.

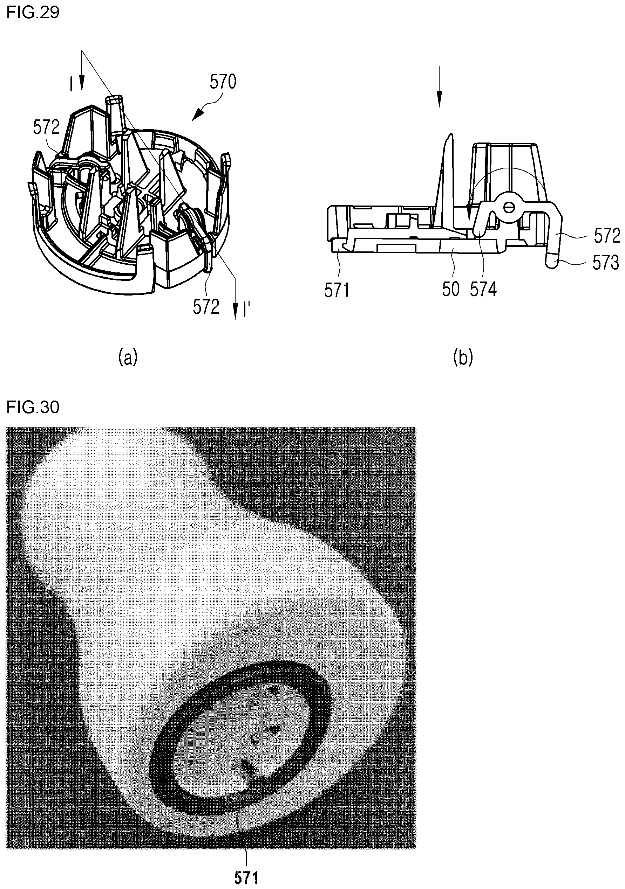

FIG. 29 illustrates a transmitter holder and a transmitter of the applicator according to the 5^{th}-1 embodiment of the present disclosure. FIG. 30 illustrates a state after firing of the applicator according to the 5^{th}-1 embodiment of the present disclosure is performed.

Referring to FIG. 29, the transmitter holder 570 may include a transmitter support portion 571.

(a) of FIG. 29 is a perspective view of the transmitter holder 570, and (b) of FIG. 29 is a cross-sectional view of (a) of FIG. 29 taken along a line I-I'.

The transmitter support portion 571 is formed on one surface of the transmitter holder 570, and is configured to hold and support the transmitter 50. The transmitter support portion 571 according to the present embodiment may be an o-ring (see FIG. 30). In this case, a frictional force may be formed between the o-ring and the transmitter 50, and even though there is no additional fastening or fastening releasing structure, the transmitter 50 may be stably held so that manufacturing cost can be reduced.

Meanwhile, since a bonding force due to friction is formed between the transmitter support portion 571 and the transmitter 50, a structure capable of pushing the transmitter 50 in the proximal direction is required. To this end, the transmitter holder 570 may further include a pusher 572.

The pusher 572 is disposed on an upper surface of the transmitter holder 570 and is configured to rotate about an axis parallel to the horizontal plane (see (b) of FIG. 29). Meanwhile, in order to provide a sufficient pressing force, preferably, the pusher 572 is provided in plurality.

A first end 573 of the pusher 572 is disposed outside the transmitter housing 510, and a second end 574 is disposed inside the transmitter holder 570. While the transmitter holder 570 is pressed (an arrow direction of (b) of FIG. 29), the first end 573 collides with the inner bottom of the housing 510. Thus, the pusher 572 may rotate in one direction. Here, one direction means a direction illustrated by the curve arrow in (b) of FIG. 29.

While the pusher 572 rotates, the second end 574 presses the transmitter 50 strongly. Thus, the transmitter 50 fixed by the transmitter support portion 571 may be removed from the transmitter holder 570.

### 6. 5^{th}-2 embodiment

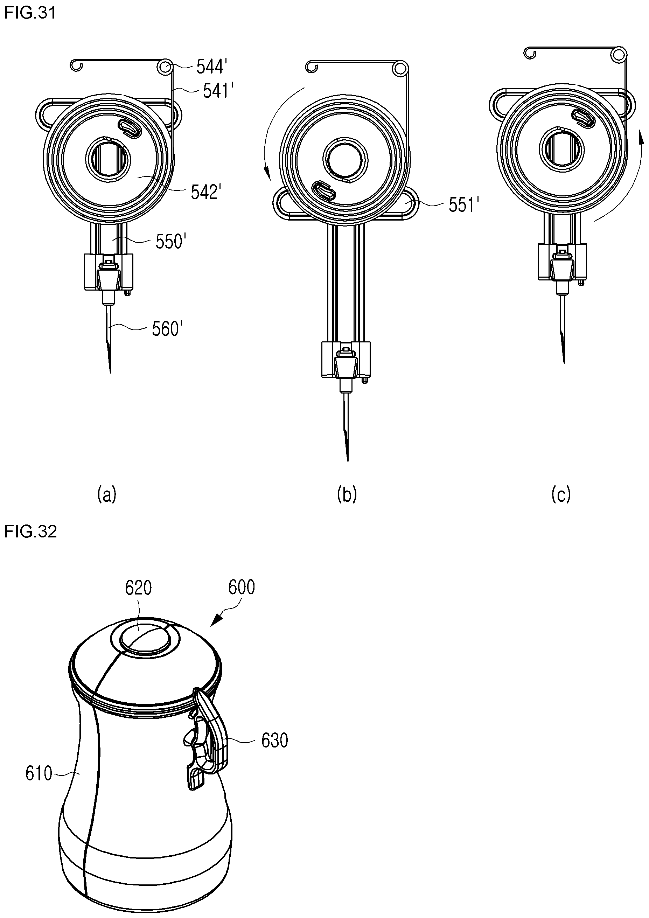

FIG. 31 illustrates the operating principle of an applicator according to a 5^{th}-2 embodiment of the present disclosure.

The applicator according to the 5^{th}-2 embodiment may share the other configuration except for a driving portion 540' with the applicator according to the 5^{th}-2 embodiment. Thus, it is noted that, in the present disclosure, only the driving portion 540' will be described.

The driving portion 540' of the applicator according to the 5^{th}-2 embodiment of the present disclosure includes an elastic body 541', a wheel 542', a guide protrusion, and all or part of a guide protrusion 544'.

The elastic body 541' has elasticity and thus is formed from a tensile material. For example, the elastic body 541' may be a rubber band, however, a material of the elastic body 541' may be properly changed by a designer.

One end of the elastic body 541' is fastened to the wheel 542', and the other end thereof is fastened to one surface of the drum. Here, one end means the end disposed inside in a state in which the elastic body 541' is wound, and the other end means the end disposed outside.

The elastic body 541' is tensioned and assembled in an initial state. By pressing of the first button 520 and the second button 530, a fastening portion that fixes tension of the elastic body 541' is released, and the elastic body 541' is compressed to return to it's original state. Thus, one end of the elastic body 541' is rotated.

The wheel 542' and a guide protrusion (not shown) according to the 5^{th}-2 embodiment have the same configuration, shape and function as the wheel 542 and the guide protrusion 543 according to the 5^{th}-1 embodiment and thus, detailed descriptions thereof are replaced with descriptions of the 5^{th}-1 embodiment.

The guide roller 544' protrudes from the inside surface of the housing, and may be disposed adjacent to the wheel 542' so that the elastic body 541' may be held on the guide roller 544'. Preferably, the guide roller 544' has an approximately cylindrical shape. Thus, the elastic body 541' may slide along the outer circumferential surface of the guide roller 544'.

(a) of FIG. 31 illustrates a state in which any one or more of the first button 520 and the second button 530 are not pressed, i.e., an initial state.

Referring to (a) of FIG. 31, the guide protrusion (not shown0 may be disposed in the most distal position, and thus, a needle carrier 550' may also be disposed in the distal position. Meanwhile, the elastic body 541' in this case may be in a tensile state.

Meanwhile, according to (a) of FIG. 31, since any one or more of the first button 520 and the second button 530 are not pressed, the proximal motion of the needle carrier 550' is limited by the first button 520 or the second button 530. Thus, the motions of the guide protrusion, the wheel 542', and the elastic body 541' directly/indirectly fastened to the needle carrier 550' are limited.

(b) of FIG. 31 illustrates a state in which both the first button 520 and the second button 530 are pressed and the needle 560 is inserted into the skin.

Referring to (b) of FIG. 31, while both the first button 520 and the second button 530 are pressed, fastening between the first button 520 and the second button 530 and the needle carrier 550' is released. Thus, the motion of the needle carrier 550' from the distal position to the proximal position is not limited any more. Thus, the motions of the guide protrusion, the wheel 542', and the elastic body 541' directly/indirectly fastened to the needle carrier 550' are possible.

The elastic body 541' rotates in one direction (illustrated counterclockwise in FIG. 31 of the present disclosure, however, the opposite direction is also acceptable). However, to allow rotation in the opposite direction, the direction in which the elastic body 541' is disposed on the guide roller 544', and the like need to be changed.

The guide protrusion is disposed at the most proximal position, and thus, the needle carrier 550' is also disposed at the proximal position. Meanwhile, the elastic body 541' may be in a state in which the tension of about the half of the elastic body 541' is released.

While the needle carrier 550' moves to the proximal position, the needle 560' may move to the proximal position and may be inserted into the user's skin.

(c) of FIG. 31 illustrates a state in which the needle 560' is drawn out from the user's skin.

Referring to (c) of FIG. 21, the elastic body 541' further rotates in one direction.

Thus, the guide protrusion is disposed at the distal position again and thus, the needle carrier 550' is also disposed at the distal position. Meanwhile, the tension of the elastic body 541' is fully released, and driving force is not generated by the elastic body 541'.

While the needle carrier 550' moves to the distal position again, the needle 560' may move to the distal position and may be drawn out from the user's skin.

### 7. 6^{th}-1 embodiment

FIG. 32 is a perspective view of an applicator according to a 6^{th}-1 embodiment of the present disclosure.

Referring to FIG. 32, an applicator 600 according to the 6^{th}-1 embodiment of the present disclosure includes a housing 610.

The housing 610 is configured to be grasped by a user, and preferably, has a hollow column shape. Also, the middle of the housing 610 may have a concave shape so that the user may grasp the entire housing 610. Meanwhile, the applicator 600 according to the 6^{th}-1 embodiment of the present disclosure operates in an automatic firing method, by which the applicator 600 is automatically fired when pressing a button rather than the user's direct pressing, unlike in the first through fourth embodiments described above.

To this end, the applicator 600 further includes a button 620.

The button 620 is disposed on the upper surface of the housing 610 and is configured to be pressed downward.

Meanwhile, in order to prevent the applicator 600 from accidental firing by pressing of the button 620 due to external impact or the like regardless of the user's intention, the applicator 600 may further include a safety pin 630.

The safety pin 630 is configured to be inserted into or removed from the housing 610, and it is preferable that the safety pin 630 includes a knob-shaped configuration so that the user may grasp the safety pin 630 easily.

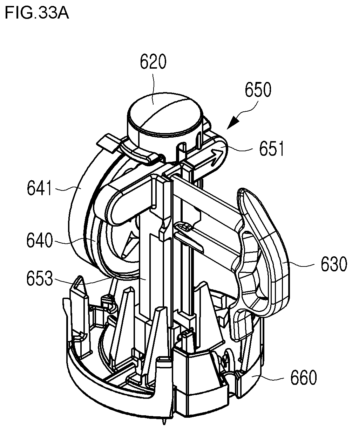

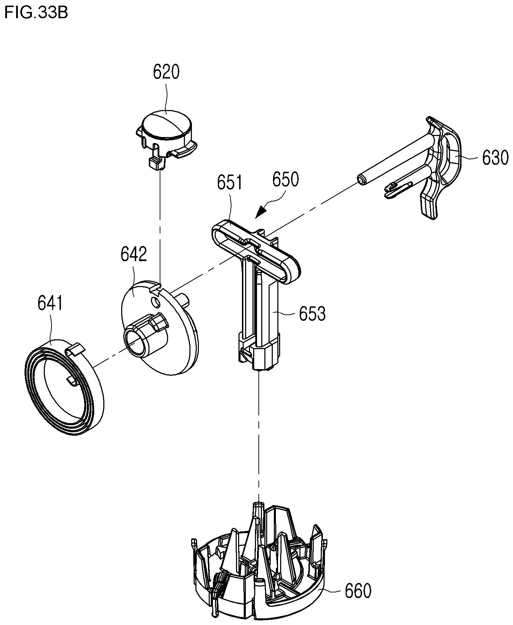

FIGS. 33A and 33B illustrate the inside of the applicator according to the 6^{th}-1 embodiment of the present disclosure.

Specifically, FIG. 33A is a rear perspective view of a state in which internal components are coupled to each other, and FIG. 33B is a front exploded perspective view of a state in which internal components are disassembled.

The combination relationship between components of the applicator 600 according to the 6^{th}-1 embodiment of the present disclosure will be described with reference to FIGS. 33A and 33B.

The applicator 600 according to the 6^{th}-1 embodiment includes a driving portion 640, a needle carrier 650, and all or part of a transmitter holder 660, which are arranged inside the housing 610.

The driving portion 640 includes a spring 641 and a wheel 642.

The spring 641 and the wheel 642 according to the 6^{th}-1 embodiment have the same configuration, shape and function as the spring 541 and the wheel 542 of the applicator 500 according to the 5^{th}-1 embodiment and thus, detailed descriptions thereof are replaced with descriptions of the 5^{th}-1 embodiment.

The needle carrier 650 is configured to make a straight line motion inside the housing 610. In this case, the straight line motion of the needle carrier 650 may be induced by the rotation motion of the driving portion 640. To this end, the needle carrier 650 may include a lateral guide 651 and a longitudinal guide 653, and the needle carrier 650 may have an overall shape of the English letter T.

A guide protrusion (not shown) of the driving portion 640 may be inserted into the lateral guide 651. When the wheel 642 rotates, the guide protrusion may rotate, and thus, the guide protrusion may move left and right along the lateral guide 651. Thus, the needle carrier 650 makes a straight line motion between the distal position and the proximal position.

The lateral guide 651 is configured to be fastened to the button 620, and firing is possible only when fastening with the button 620 is released. Since the fastening relationship between the first button 520 and the lateral guide 551 according to the 5^{th}-1 embodiment of the present disclosure may be applied to fastening of the button 620 and the lateral guide 651 according to the present embodiment, descriptions thereof will be replaced with descriptions of the 5^{th}-1 embodiment.

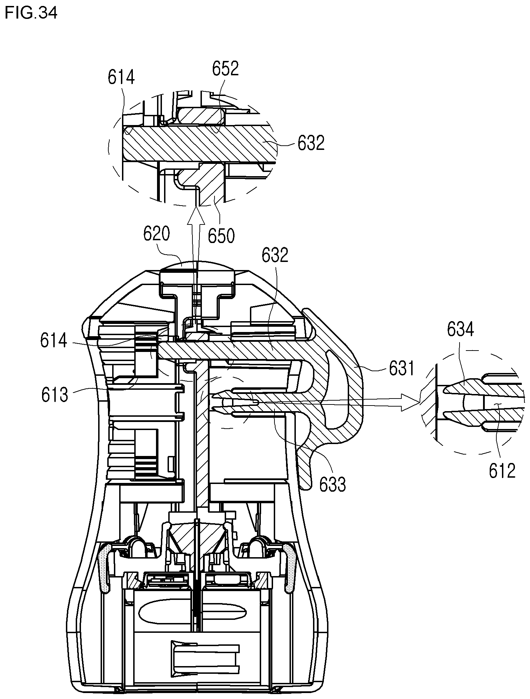

The longitudinal guide 653 is configured to be fastened to the safety button 630, and is movable only when fastening with the safety button 630 is released. In this regard, this will be described in detail with reference to FIG. 34.

The transmitter holder 660 is coupled to a transmitter (not shown) and is configured to make a straight line motion of the transmitter from the distal position to the proximal position. The transmitter holder 660 is pressed from the distal position to the proximal position by the longitudinal guide 650, and the transmitter holder 660 moves only in a direction from the distal position to the proximal position.

FIG. 34 is a cross-sectional view of FIG. 32 taken along a line I-I' and an expanded view thereof.

Referring to FIG. 34, a safety pin 630 may include a gripping part 631, a first pin 632, and a second pin 633.

The gripping part 631 is a part grasped by the user.

The first pin 632 protrudes from the gripping part 631 and preferably, has a thin pin shape.

The second pin 633 protrudes from the gripping part 631 and is disposed below the first pin 632. In this case, directions in which the first pin 632 and the second pin 633 protrude, are the same.

The first pin 632 and the second pin 633 are inserted into one surface of the housing 610, and firing is prevented by the first pin 632 and the second pin 633.

A hook 634 may be formed on the end of the second pin 633. The hook 634 is hung on the end of a second pin hole 612 so that the safety pin 630 can be prevented from being removed from the applicator 600 due to external impact or the like. Meanwhile, an inclined surface is formed on the hook 634 in a direction in which the safety pin 630 is removed from the applicator 600. Thus, the safety pin 630 may be removed from the applicator 600 only when the safety pin 630 is pulled by a certain force or more. In other words, the hook 634 may be coupled to the second pin hole 612 in a snap fit manner.

The housing 610 may include a first pin hole 611, a second pin hole 612, a spring mounting portion 613, and a first pin groove 614.

The first pin hole 611 is a hole formed in the outer circumferential surface of the housing 610, and the first pin 632 may be inserted into the first pin hole 611.

The needle carrier 650 may include a first pin through hole 652.

The first pin through hole 652 is formed in the center of the lateral guide 651 of the needle carrier 650, and the first pin 632 may perforate the needle carrier 650 through the first pin through hole 652.

The spring mounting portion 613 is a configuration in which the spring 641 is supported, and may have an entirely boss shape. The first pin groove 614 is formed in a flange part of the spring mounting portion 613.

The first pin hole 611, the first pin through hole 652, and the first pin groove 614 described above have circular shapes with the same diameter, and are arranged axially. Thus, the first pin 632 allows all of the housing 610, the needle carrier 650, and the driving portion 640 to be fixed at one time.

Meanwhile, in a state in which the first pin 632 is inserted, the end of the first button 620 is in contact with the first pin 632. Thus, as long as the first pin 632 is inserted, the first button 620 may not be pressed.

The second pin hole 612 is a hole formed in the outer circumferential surface of the housing 610, and the second pin 633 is inserted into the second pin hole 612, and the second pin hole 612 is formed below the first pin hole 611.

In the applicator 600 according to the 6^{th}-1 embodiment, when the safety pin 630 is inserted, pressing of the button 620 can be prevented, and the rotation of the driving portion 640 and the straight line motion of the needle carrier 650 can also be prevented.

In addition, by using the hook 634 of the safety pin 630, a safety pin is prevented from being taken off due to external impact so that safety can be further secured.

### 8. 6^{th}-2embodiment

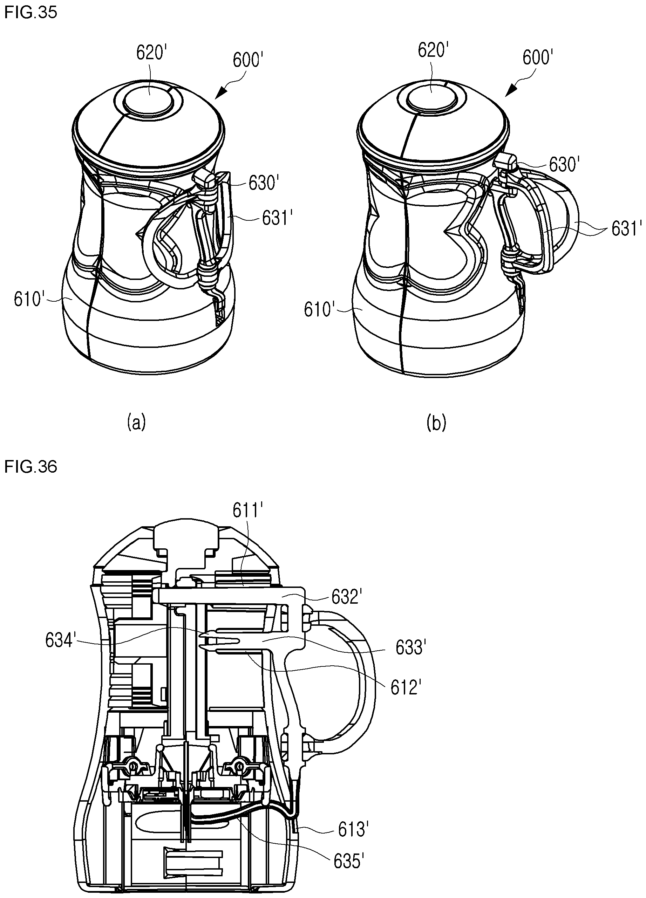

FIG. 35 is a perspective view of an applicator according to a 6^{th}-2 embodiment of the present disclosure.

(a) of FIG. 35 illustrates a state in which a gripping part 631' is unfolded, and (b) of FIG. 35 illustrates a state in which the gripping part 631' is gathered.

Referring to FIG. 35, an applicator 600' according to the 6^{th}-2 embodiment of the present disclosure includes a housing 610', a button 620', and all or part of a safety pin 630'.

The housing 610' and the button 620' according to the 6^{th}-2 embodiment have the same configuration, shape, and function as those of the housing 610 and the button 620 according to the 6^{th}-1 embodiment and thus, descriptions thereof will be replaced with descriptions according to the 6^{th}-1 embodiment.

The safety pin 630' according to the 6^{th}-2 embodiment has the gripping part 631' including two configurations, and the gripping part 631' may be unfolded or gathered. The gripping part 631' is unfolded so that the entire volume of the applicator 600' can be reduced and thus reduction of packaging materials is possible.

FIG. 36 is a cross-sectional perspective view of the applicator according to the 6^{th}-2 embodiment of the present disclosure.

Referring to FIG. 36, the safety pin 630' according to the 6^{th}-2 embodiment of the present disclosure may further include a first pin 632', a second pin 633', a hook 634', and a support pin 635'.

The first pin 632', the second pin 633', and the support pin 635' are arranged in order along the height direction of the housing 610'.

The first pin 632' is inserted into the first pin hole 611', the second pin 633' is inserted into the second pin hole 612', and the support pin 635' is inserted into a support pin hole 613'.

The configuration, shape, and function of the first pin 632', the second pin 633', and the hook 634' are the same as those of the first pin 632, the second pin 633, and the hook 634 according to the 6^{th}-1 embodiment and thus, descriptions thereof will be replaced with descriptions of the 6^{th}-1 embodiment.

The support pin 635' is in contact with the lower surface of a transmitter in a state in which the safety pin 630' is inserted into the housing 610', so that the transmitter and a transmitter holder can be prevented from moving to the proximal position.

### 9. Seventh embodiment

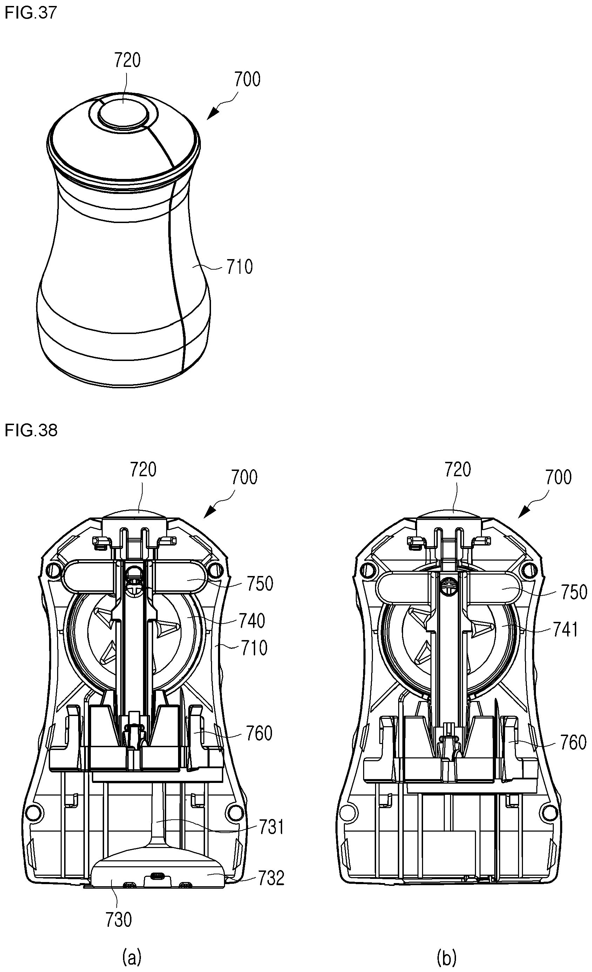

FIG. 37 is a perspective view of an applicator according to a seventh embodiment of the present disclosure.

Referring to FIG. 37, an applicator 700 according to the seventh embodiment of the present disclosure includes a housing 710. The housing 710 is configured to be grasped by the user, and preferably, has a hollow column shape. Also, the middle of the housing 710 may have a concave shape so that the user may grasp the entire housing 710. Meanwhile, the applicator 700 according to the seventh embodiment of the present disclosure operates in an automatic firing method, by which the applicator 500 is automatically fired when pressing a button rather than the user's direct pressing, unlike in the first through fourth embodiments described above.

To this end, the applicator 700 further includes a button 720.

The button 720 is disposed on the upper surface of the housing 710 and is configured to be pressed downward.

FIG. 38 illustrates the inside of the applicator according to the seventh embodiment of the present disclosure.

Referring to FIG. 38, the applicator 700 according to the seventh embodiment of the present disclosure includes a cap 730, a driving portion 740, a needle carrier 750, and all or part of a transmitter holder 760.

The cap 730 is formed so that at least a part of the cap 730 may be inserted through an opening formed in the bottom of the housing 710. The cap 730 is configured to protect the inside of the applicator 700 from external contaminants before the user uses the applicator 700. To this end, it is preferable that the maximum diameter of the cap 730 is the same as or slightly greater than the diameter of the opening formed in the bottom of the housing 710.

Although the cap 730 may be formed of one of an acrylonitrile butadiene styrene copolymer (ABS) resin, a polycarbonate (PC) resin, and a polyethylene (PE) resin, any material generally used in a medical device such as an applicator may be used without limitations. In an example, the PC resin and the ABS resin may be combined with each other and used, however, this may be properly changed by the designer's selection.

The cap 730 may include a support rod 731 and a sealing portion 732. The support rod 731 is a configuration that is in contact with a transmitter and supports the transmitter and the transmitter holder 760 directly.

The sealing portion 732 is configured to seal the opening formed at the bottom of the housing 710.

The driving portion 740 is configured to make a straight line motion of the needle carrier 750 and a needle (not shown) between the distal position and the proximal position in response to pressing of the button 720. To this end, the driving portion 740 may include a wheel 741. Meanwhile, the wheel 741 according to the seventh embodiment has the same configuration, shape and function as those of the wheel 542 of the applicator 500 according to the 5^{th}-1 embodiment and thus, descriptions thereof will be replaced with descriptions of the 5^{th}-1 embodiment.

The needle carrier 750 is configured to make a straight line motion inside the housing 710. In this case, the straight line motion of the needle carrier 750 may be induced by the rotation motion of the driving portion 740.

The transmitter holder 760 is coupled to a transmitter (not shown) and is configured to make a straight line motion of the transmitter from the distal position to the proximal position. The transmitter holder 760 is pressed by the needle carrier 750 from the distal position to the proximal position. Meanwhile, the transmitter holder 760 moves only in a direction from the distal position to the proximal position.

(a) of FIG. 38 illustrates a state before the cap 730 is removed, and (b) of FIG. 38 illustrates a state in which the cap 730 has been removed.

Referring to (a) of FIG. 38, the cap 730 supports the transmitter holder 760 in a direction toward the distal position. Thus, a configuration capable of making a straight line motion, i.e., the needle carrier 760 is also supported in a direction toward the distal position.

Referring to (b) of FIG. 38, when the cap 730 is removed, the needle carrier 750 and the transmitter holder 760 may be slightly lowered toward the proximal position. In this state, the user may press the button 720, and the applicator 700 is ready to fire.

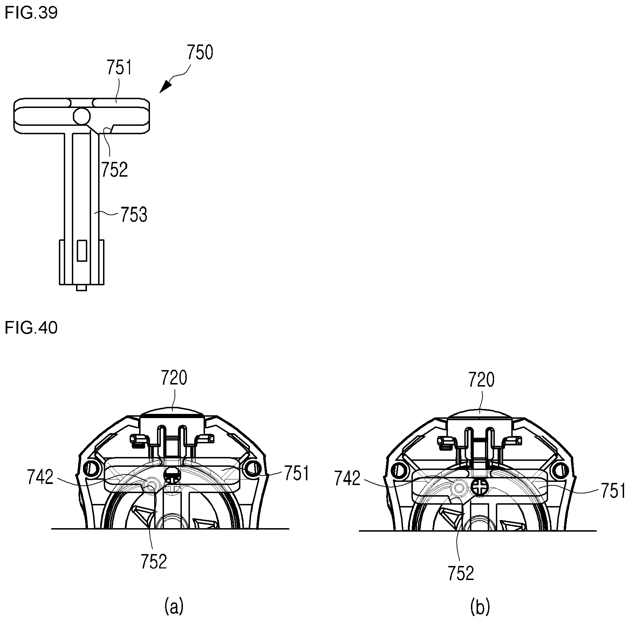

FIG. 39 is a front view of a needle carrier according to the seventh embodiment of the present disclosure.

Referring to FIG. 39, the needle carrier 750 of the present disclosure includes a lateral guide 751, an insertion groove 752, and a longitudinal guide 753.

A guide protrusion (see 742 of FIG. 40) of the driving portion 740 may be inserted into the lateral guide 751. While the wheel 741 rotates, the guide protrusion 742 rotates, and thus, the guide protrusion 742 may move left and right along the lateral guide 751. Thus, the needle carrier 750 makes a straight line motion between the distal position and the proximal position.

The insertion groove 752 is formed in the bottom of the lateral guide 751, and preferably, has a width at which the guide protrusion 742 passes through the insertion groove 752.

The longitudinal guide 753 extends from one surface of the lateral guide 751 in a direction from the distal position to the proximal position. The longitudinal guide 753 may be coupled to a needle and fix the needle.

FIG. 40 illustrates a configuration for preventing firing of the applicator according to the seventh embodiment of the present disclosure.

(a) of FIG. 40 illustrates a state before the cap 730 is removed, and (b) of FIG. 40 illustrates a state in which the cap 730 has been removed.

Referring to (a) of FIG. 40, since the needle carrier 750 is supported at the distal position before the cap 730 is removed, the needle carrier 750 moves slightly to the distal position. Thus, the guide protrusion 742 may be positioned over the insertion groove 752 of the needle carrier 750. Thus, the lateral guide 751 may not move laterally. Meanwhile, the lateral movement of the guide protrusion 742 is mutually constrained to the longitudinal movement due to the nature of circular motion, and lateral movement is not possible so that the longitudinal movement of the guide protrusion 742 is also limited. Thus, firing can be prevented.

Referring to (b) of FIG. 40, when the cap 730 is removed, a configuration for supporting the needle carrier 750 is removed, so that the needle carrier 750 moves slightly to the proximal position. The guide protrusion 742 is inserted into the inside of the lateral guide 751, and thus, lateral movement is possible.

While the above description is merely exemplary description of the technical spirit of the present embodiment, it will be understood by those skilled in the art that various modifications and changes can be made to the present disclosure without departing from the essential characteristics of the present embodiment. Therefore, the present embodiments are not intended to limit the technical spirit of the present embodiment, but to describe the technical spirit of the present embodiment, and the scope of the technical spirit of the present embodiment is not limited by this embodiment. The protection scope of the present embodiment should be interpreted by the following claims, and all technical spirits within the equivalent scope should be interpreted to be included in the scope of rights of the present embodiment.

### [Explanation of reference numerals]

100, 200, 300, 400, 500, 600, 600', 700: applicator
110, 210, 410: handle
120, 220, 420, 730: cap
130, 230: support portion
140: gear
150, 260, 550, 550', 650, 750: needle carrier
160: needle holder
170, 270, 362, 560, 560': needle
180, 280, 380, 450, 570, 660, 760: transmitter holder
240: crank
250: connecting rod
310: upper cap
312: lower cap
330: push cap
340: rotation guide
350: rotation block
370: spring
430: cylinder
440: piston
520: first button
530: second button
540, 540', 640, 740: driving portion
620, 620', 720: button
630, 630': safety pin

## Claims

1. An applicator comprising:
a hollow column-shaped housing having an opening formed in a bottom thereof;
a first button disposed on an upper surface of the housing;
a second button disposed on an outer circumferential surface of the housing;
a needle carrier configured to be fastened to the second button and making a straight line motion between a distal position and a proximal position inside the housing; and
a driving portion configured to be fastened to the first button and to provide power of the straight line motion of the needle carrier.

2. The applicator of claim 1, wherein the driving portion comprises:
a wound spring in a compressed state coupled to an inner circumferential surface of the housing;
a wheel having one surface facing the spring and constrained to rotation of the spring;
an arc-shaped firing preventing portion that protrudes from the other surface of the wheel and is formed along a circumference of the wheel; and
a guide protrusion that protrudes from the other surface of the wheel and is disposed radially inward from the firing preventing portion,
wherein the needle carrier comprises:
a lateral guide; and
a longitudinal guide that is formed from one surface of the lateral guide and extends along a lengthwise direction of the housing.

3. The applicator of claim 2, wherein the first button comprises:
an extension portion that protrudes from a lower surface of the first button; and
an engagement protrusion that protrudes from one surface of the extension portion toward a radial outside of the first button and configured to face the firing preventing portion, and
the engagement protrusion is spaced apart from the lower surface of the first button, and when the first button is pressed, the firing preventing portion is disposed in the spaced space between the engagement protrusion and the lower surface of the first button.

4. The applicator of claim 2, wherein the needle carrier comprises a hook protrusion that protrudes from a lateral surface of the longitudinal guide, and the second button comprises:
an extension portion that protrudes from a lower surface of the second button and disposed toward a radially inside of the housing; and
a hook that protrudes from one surface of the extension portion toward a radial inside of the second button and configured to face the hook protrusion, and
the hook is spaced apart from the lower surface of the second button, and when the second button is pressed, the hook protrusion is disposed in the spaced space between the hook and the lower surface of the second button.

5. The applicator of claim 2, wherein the first button is configured to limit rotation of the driving portion, the second button is configured to limit a straight line motion of the needle carrier, and when the first button and the second button are simultaneously or sequentially pressed, the driving portion is rotated so that the needle carrier moves from the distal position to the proximal position.

6. The applicator of claim 1, further comprises a transmitter holder that is pressed by the needle carrier, moves from the distal position to the proximal position and configured to hold a transmitter,
wherein the transmitter holder comprises:
a transmitter support portion configured to surround at least a part of the transmitter; and
a pusher rotatably mounted on an upper surface of the transmitter holder.

7. The applicator of claim 6, wherein one end of the pusher is disposed outside the transmitter holder, the other end of the pusher is disposed inside the transmitter holder, and one end of the pusher protrudes from a lower surface of the transmitter holder.

8. The applicator of claim 7, wherein, when the transmitter holder moves from the distal position to the proximal position by the needle carrier, one end of the pusher is in contact with an inner bottom of the housing, and the other end of the pusher presses an upper surface of the transmitter.

9. The applicator of claim 6, wherein the transmitter support portion and the transmitter are frictionally coupled to each other.

10. A sensor insertion apparatus comprising:
a sensor of which at least a part is inserted into a body and which is configured to detect body analytes;
a transmitter configured to process information related to the body analytes obtained from the sensor; and
an applicator configured to move the sensor and the transmitter from a proximal position to a distal position,
wherein the applicator comprises:
a hollow column-shaped housing having an opening formed in a bottom thereof;
a first button disposed on an upper surface of the housing;
a second button disposed on an outer circumferential surface of the housing;
a needle carrier configured to be fastened to the second button and making a straight line motion between a distal position and a proximal position inside the housing; and
a driving portion configured to be fastened to the first button and to provide power of the straight line motion of the needle carrier.
